# EUROPEAN PATENT SPECIFICATION

(11) **EP 2 130 483 B1**
(45) Date of publication and mention of the grant of the patent: **19.10.2016**
(21) Application number: 09007468.3
(22) Date of filing: 05.06.2009
(51) Int. Cl.: A61B 1/00, A61B 1/04, A61B 1/313, A61B 17/00, A61B 17/29, A61B 17/30, A61B 17/22

(54) **Medical apparatus**
Medizinisches Gerät
Appareil médical

(30) Priority: 06.06.2008 JP 2008149750; 19.01.2009 JP 2009009249
(43) Date of publication of application: 09.12.2009
(73) Proprietor: Olympus Corporation, Tokyo 192-8507 (JP)
(72) Inventor: Karasawa, Hitoshi, Tokyo 151-0072 (JP); Yazawa, Nobuyoshi, Tokyo 151-0072 (JP); Nakajima, Sho, Tokyo 151-0072 (JP); Urakawa, Tsutomu, Tokyo 151-0072 (JP); Asada, Daisuke, Tokyo 151-0072 (JP)
(74) Representative: von Hellfeld, Axel

(56) References cited:
- EP-A- 1 902 663
- EP-A- 2 082 681
- WO-A-98/11816
- WO-A-2009/037880
- WO-A1-2007/040269
- JP-A- 2005 237 979
- US-A1- 2005 165 272
- US-A1- 2007 255 100
- US-A1- 2008 015 413
- US-A1- 2008 058 835
- RENTSCHLER M E ET AL: "Recent in vivo surgical robot and mechanism developments" SURGICAL ENDOSCOPY ; AND OTHER INTERVENTIONAL TECHNIQUES OFFICIAL JOURNAL OF THE SOCIETY OF AMERICAN GASTROINTESTINAL AND ENDOSCOPIC SURGEONS (SAGES) AND EUROPEAN ASSOCIATION FOR ENDOSCOPIC SURGERY (EAES), SPRINGER-VERLAG, NE, vol. 21, no. 9, 19 May 2007 (2007-05-19), pages 1477-1481, XP019543229 ISSN: 1432-2218
- OLEYNIKOV D ET AL: "Miniature robots can assist in Laparoscopic cholecystectomy" SURGICAL ENDOSCOPY ; AND OTHER INTERVENTIONAL TECHNIQUES OFFICIAL JOURNAL OF THE SOCIETY OF AMERICAN GASTROINTESTINAL AND ENDOSCOPIC SURGEONS (SAGES) AND EUROPEAN ASSOCIATION FOR ENDOSCOPIC SURGERY (EAES), SPRINGER-VERLAG, NE, vol. 19, no. 4, 1 April 2005 (2005-04-01), pages 473-476, XP019366598 ISSN: 1432-2218

## Description

### BACKGROUND OF THE INVENTION

### 1. Field of the Invention

The present invention relates to a medical apparatus having a medical device that is secured to an inner surface of abdominal wall.

### 2. Description of Related Art

As is well known, an endoscope is a medical device having an image pickup apparatus, and when the endoscope is introduced in a patient's body cavity, the image pickup apparatus picks up observation images of the cavity, so that the images are used in various tests and various treatments for diseased parts in the body.

Such endoscopes are classified into two groups: one is composed of those that are introduced in alimentary organs such as esophagus, stomach, large intestine, and duodenum that are tube cavities and tracts in body from anus or oral cavity of the body; and the other is composed of those that are introduced in abdominal cavity inserted through a body wall at a position near umbilicus of the body. Generally, an endoscope includes an elongated insertion portion which is inserted into a tract of a digestive organ or an abdominal cavity.

In recent years, in order to reduce pain in patient in the introduction of such an insertion portion, for example, capsule-type medical apparatuses described in Japanese Patent Application Laid-Open Publication No. 2006-271492, Japanese Patent Application Laid-Open Publication No. 2003-530135, and Japanese Patent Application Laid-Open Publication No. 2007-89893 have been proposed.

Japanese Patent Application Laid-Open Publication No. 2006-271492 discloses a capsule-type in-vivo inspection apparatus including an LED for illumination and an image pickup element that are mounted to a circuit board and fixed to the inside of the capsule. The apparatus has a cam member arranged to be rotated between a pair of actuation rods arranged in the direction orthogonal to the center line with respect to a fitting block of the circuit board.

Japanese Patent Application Laid-Open Publication No. 2003-530135 discloses an implantable monitoring probe having a capsule-type endoscope in which an attachment cavity to be attached to a body wall relative to image pickup means, and a grasp portion configured to be grasped.

Moreover, Japanese Patent Application Laid-Open Publication No. 2007-89893 discloses a placement system of introduction device in body cavity that includes holding means configured to hold a capsule-type endoscope, tissue coupling means configured to secure the endoscope to tissues in body cavity by piercing a clip through the tissues for clipping, and a placement device configured to control the operation of the capsule-type endoscope.

However, the capsule-type endoscopic devices described in Japanese Patent Application Laid-Open Publication No. 2006-271492, Japanese Patent Application Laid-Open Publication No. 2003-530135, and Japanese Patent Application Laid-Open Publication No. 2007-89893 are based on the technology that is effective in tube cavities and tracts in body, but the technology cannot be readily applied to the observation with an endoscope in abdominal cavity.

In other words, the conventional manipulation for introducing an endoscope in abdominal cavity involves a surgery which is so-called laparoscopic surgery that includes, for example, inserting a trocar to introduce an endoscope for observation into a body cavity and another trocar to introduce a treatment instrument to a region for treatment into patient's abdominal parts, and treating the region while the treatment instrument and the region for treatment are observed by the endoscope, without significant laparotomy to make the manipulation minimally invasive. The approach provides an advantage that a region for treatment can be observed in detail by an endoscope, but has a problem that the obtained visual range for observation is relatively narrow. Thus, in using the approach, in addition to a normal endoscope, preferably an image pickup apparatus such as an endoscope for wide-angle observation that provides a wider visual range is simultaneously used so that the entire region for treatment in abdominal cavity is widely observed.

However, the laparoscopic surgery involving the insertion of an endoscope for wider observation in abdominal cavity in addition to a normal endoscope through trocars that are inserted in patient's abdominal wall that is a body wall requires the insertion of a plurality of trocars in the patient's abdominal wall. This increases burden to the patient, and makes the laparoscopic surgery not minimally invasive.

In addition, such a capsule-type endoscopic apparatus includes various devices for image pickup, illumination, and power source arranged therein. Thus, such devices are preferably effectively arranged in a limited space of a capsule-type endoscopic apparatus. Also, when used in laparoscopic surgery, such a capsule-type endoscopic apparatus having a large outer size requires a trocar having a large size to be introduced in abdominal cavity, as the result of that insertion for the trocar involves significant laparotomy, which makes the laparoscopic surgery not minimally invasive.

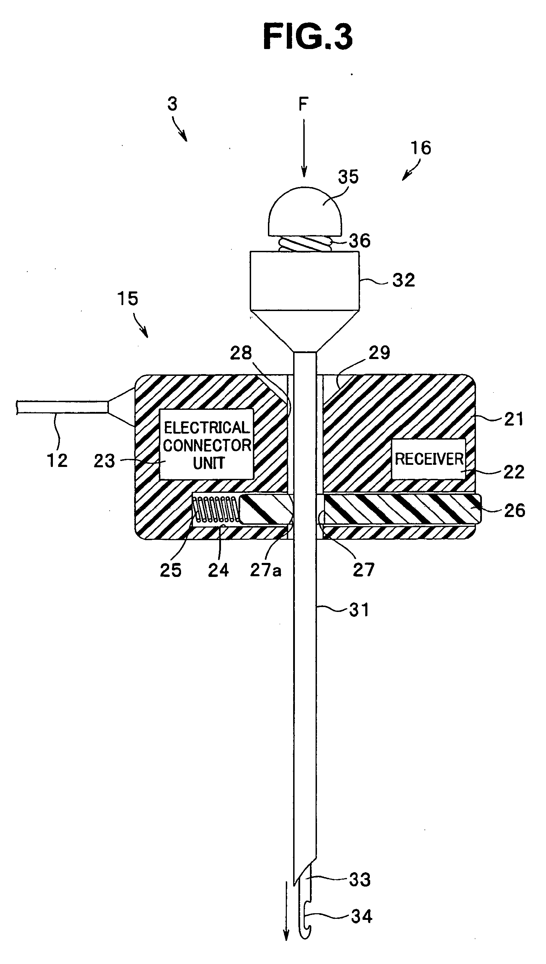

Rentschler, M. E., et al., "Recent in vivo surgical robot and mechanism developments", Surgical Endoscopy, Springer Science+Business Media, LLC 2007, vol. 21, no. 9, 19 May 2007, pgs. 1477-1481 discloses an in vivo surgical robot for laparoscopic surgery comprising a cylindrical shaped body, retractable tripod legs extending from a base of the cylinder, a handle portion disposed at an opposite side of the cylinder from the legs, an LED device and a camera device disposed on the camera. The camera lens is disposed on an outer portion of the cylinder, and the robot is disposed in a bodily cavity with the legs forming a means of fixing the robot to the abdominal wall, wherein a tilting mechanism attached to a central portion of the cylinder is adapted to allow the robot to tilt up to a 45° angle within the abdominal cavity (Figs. 3-4).

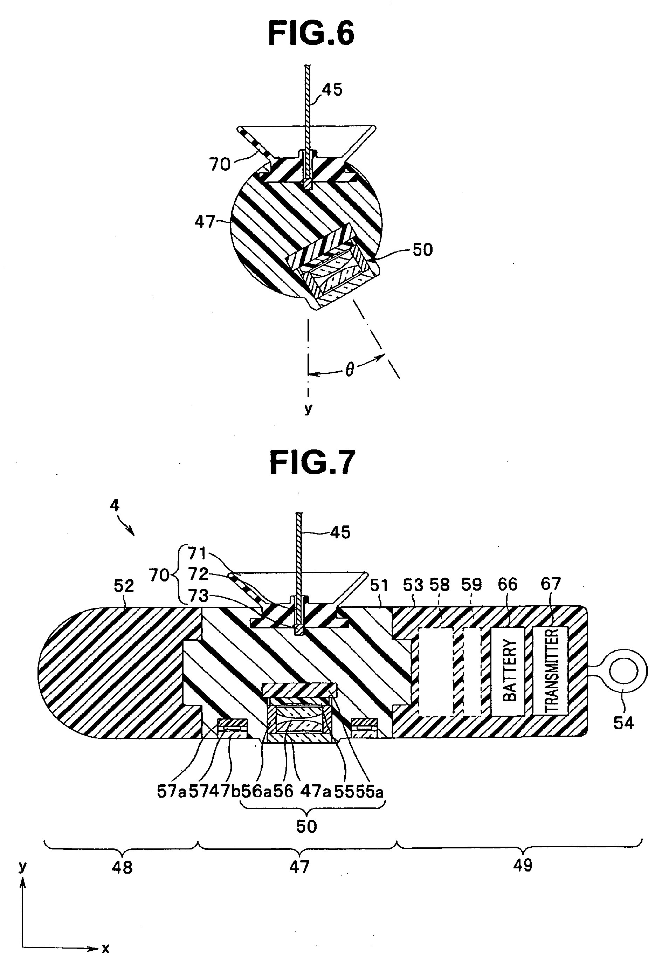

EP 1 902 663 discloses a capsule-type medical device having an image pickup device with an optical axis arranged in parallel to a longitudinal axis of the capsule-type medical device. Several means of attaching the capsule-type medical device to body tissue comprising handles attached to an outer portion of a longitudinal section of the capsule-type medical device, or, alternatively, a net apparatus for holding the capsule-type medical device to a bodily wall, are disclosed (Figs. 6 and 15 to 39).

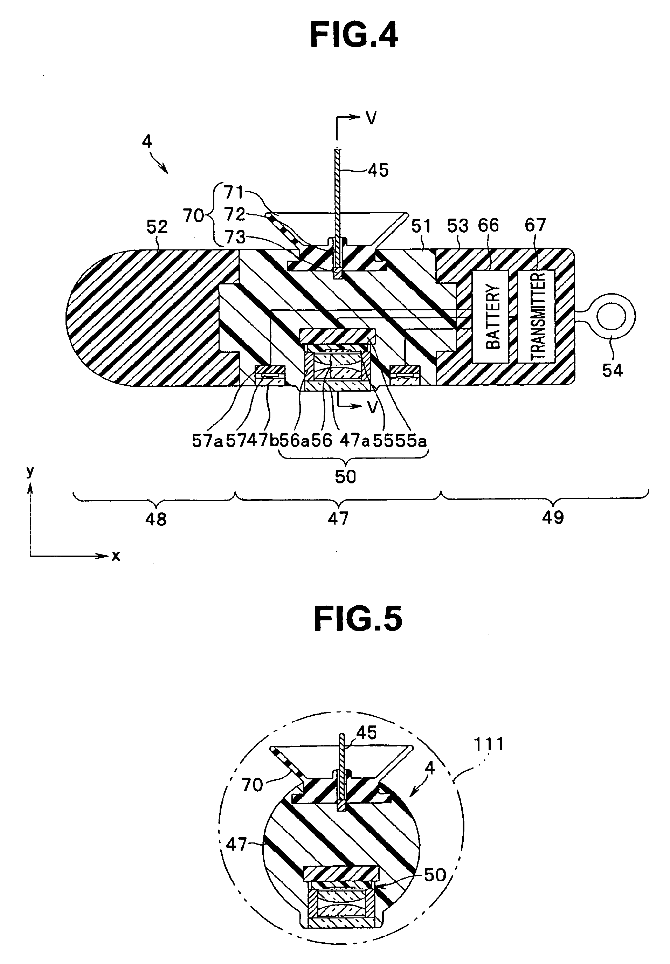

US 2007/255100 discloses a capsule-type medical device having an image pickup pickup device with an optical axis arranged in parallel to a longitudinal axis of the capsule-type medical device. The capsule device is adapted to receive an end of an instrument controlled outside the body cavity, which is moved to provide alternative imaging viewing directions. The instrument may be received on an end thereof, opposite to the end on which the image pickup unit is disposed, or on an outer peripheral portion of the capsule device. (Figs. 5, 25, and 29).

WO 98/11816 discloses an apparatus for imaging an in vivo location comprising an imaging unit having an optical axis along a longitudinal direction of the apparatus (Fig. 4).

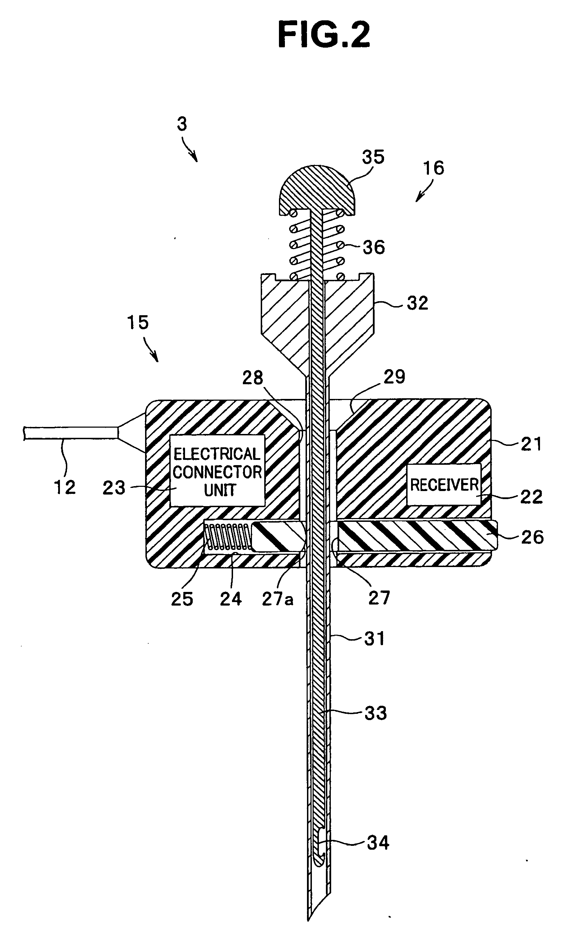

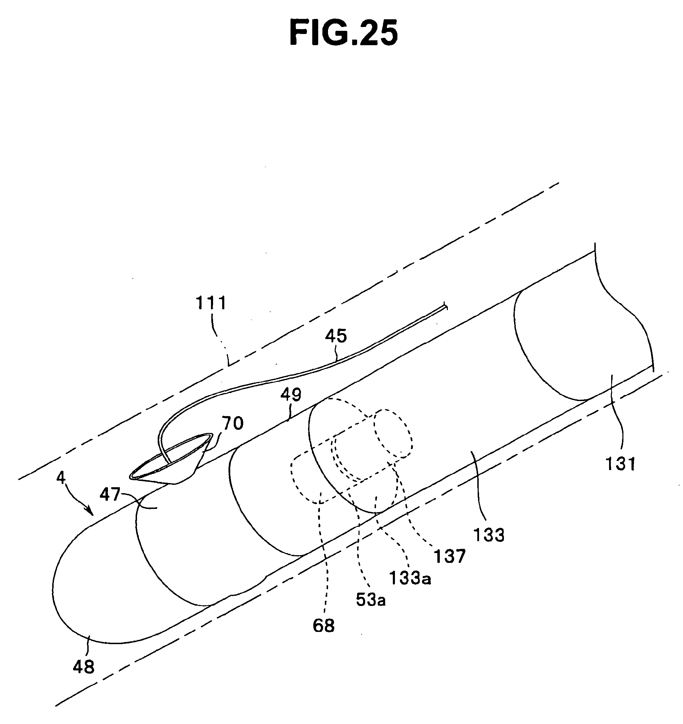

US 2005/1652752 discloses a capsule endoscope having an observation optical system having an optical axis along a longitudinal axis of the capsule endoscope. The capsule endoscope is adapted to be fixed to a body wall at an end portion thereof opposite to an end portion on which the observation optical system is disposed (Figs. 2, 25).

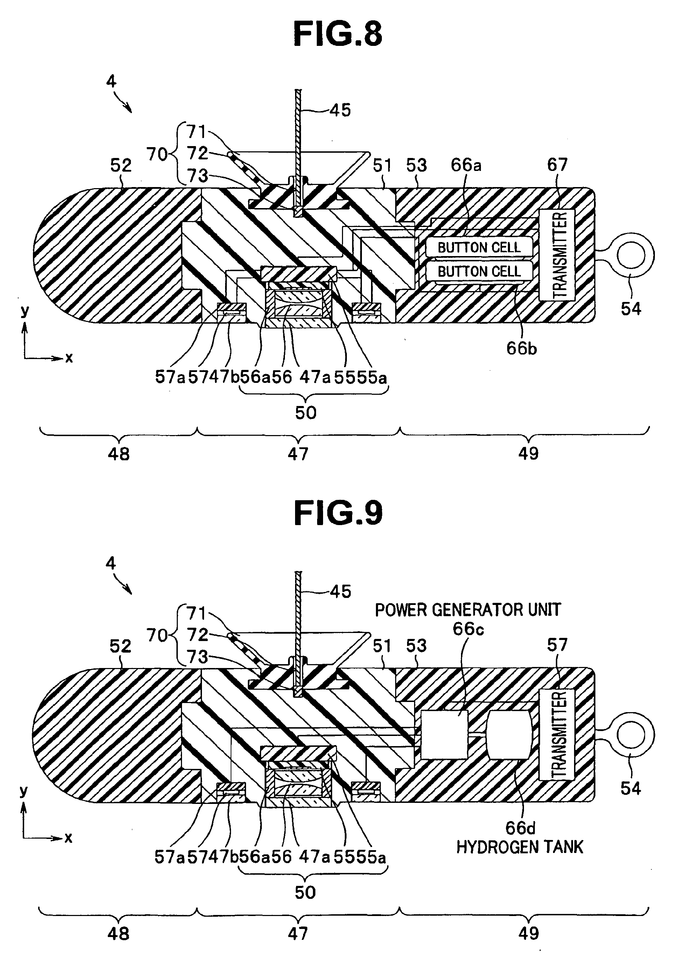

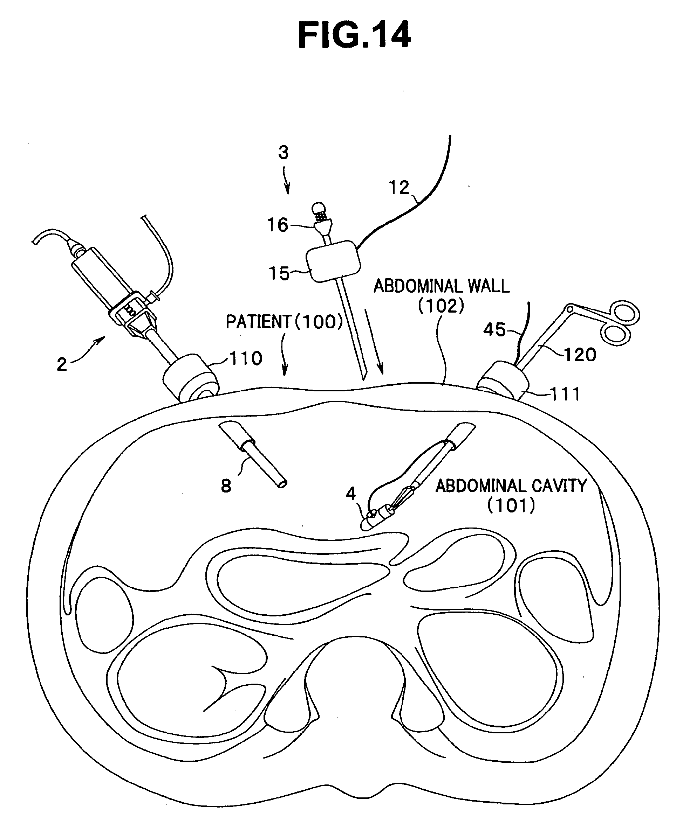

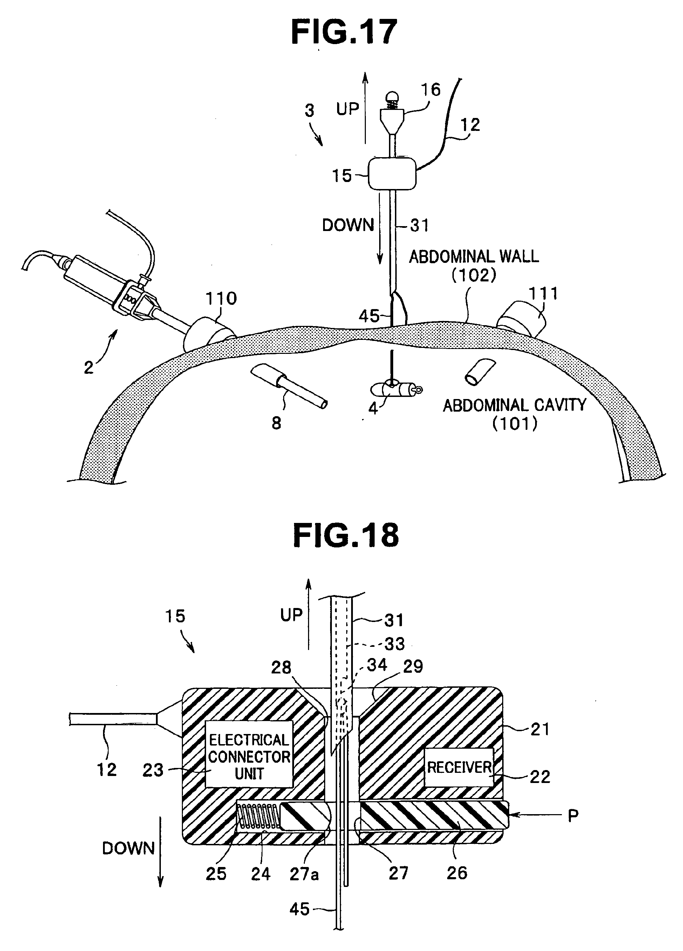

WO 2009/037880 and EP 2 082 681 (D7) disclose a capsule endoscope having an observation optical system having an optical axis along a longitudinal axis of the capsule endoscope (Fig. 17, D7, Fig. 8). US 2008/058835 shows various embodiments of modular imaging robots, in particular embodiment of Fig.9, orientable camera tilting up to 360° in Fig. 14-15 or magnetically coupled imaging robots as in Fig. 17 or Fig. 23, 25, however none having a detachable securing means having a wire inserted and hold by an external securing unit to secure the camera to the body wall.

The present invention was made in view of the above problems, and one object of the present invention is to provide a downsized medical apparatus that realizes minimally invasive surgery and especially improves the introducibility of the device into abdominal cavity, without increases burden to patient.

### SUMMARY OF THE INVENTION

In order to achieve these objects, a medial apparatus according to claim 1 is provided. A medical apparatus of the present invention includes a downsized medical device which is introduced into a body, and has: an image pickup portion that is provided in the medical device and picks up an image of a subject to be examined in the body; a securing portion configured to secure the medical device to a wall in the body; and a grasping unit that is arranged in parallel to the image pickup portion, incorporates at least one assistance apparatus that assists the image pickup, and is configured to be grasped when the medical device is introduced into and retracted out of the body, so as to realize minimally invasive surgery, and especially improves the introducibility of the device into abdominal cavity, without increases burden to patient.

The above and other objects, features and advantages of the invention will become more clearly understood from the following description referring to the accompanying drawings.

### BRIEF DESCRIPTION OF THE DRAWINGS

Fig. 1 is a view showing a configuration of an endoscope system that is a medical apparatus according to a first embodiment of the present invention;
Fig. 2 is a sectional view showing a configuration of an external apparatus according to the first embodiment;
Fig. 3 is a top view showing an operation of a puncture needle of the external apparatus according to the first embodiment;
Fig. 4 is a sectional view showing a configuration of a camera to be installed in abdominal cavity according to the first embodiment;
Fig. 5 is a sectional view taken along the V-V line of the Fig. 4 according to the first embodiment;
Fig. 6 is a sectional view showing a camera to be installed in abdominal cavity of a modified example according to the first embodiment;
Fig. 7 is a sectional view showing a camera to be installed in abdominal cavity which is provided with various assist functions according to the first embodiment;
Fig. 8 is a sectional view showing a camera to be installed in abdominal cavity having a button cell as a power source portion according to the first embodiment;
Fig. 9 is a sectional view showing a camera to be installed in abdominal cavity having a hydrogen fuel cell as a power source portion according to the first embodiment;
Fig. 10 is a sectional view showing a camera to be installed in abdominal cavity having an illumination portion arranged at a grasping unit with assist functions according to the first embodiment;
Fig. 11 is a sectional view showing a camera to be installed in abdominal cavity to illustrate an internal configuration of a transmitter according to the first embodiment;
Fig. 12 is a view showing a state where trocars are inserted through patient's abdominal wall according to the first embodiment;
Fig. 13 is a perspective view showing a state where a grasp portion of a grasping unit with assist functions is grasped by grasping forceps according to the first embodiment;
Fig. 14 is a view illustrating a procedure to introduce a camera to be installed in abdominal cavity into an abdominal cavity according to the first embodiment;
Fig. 15 is a view showing a state where a hook needle is inserted through abdominal wall and a wire of a camera to be installed in abdominal cavity is engaged with the needle, and illustrating a procedure to introduce the camera to be installed in abdominal cavity into the abdominal cavity according to the first embodiment;
Fig. 16 is a view showing a state where the hook needle engaged with the wire of the camera to be installed in abdominal cavity is pulled up, and illustrating a procedure to secure the camera to be installed in abdominal cavity to the abdominal wall according to the first embodiment;
Fig. 17 is a view showing a state where the hook needle is pulled up and also a securing unit is pulled down along the hook needle, and illustrating a procedure to secure the camera to be installed in abdominal cavity to the abdominal wall according to the first embodiment;
Fig. 18 is a sectional view for illustrating an operation of the external apparatus according to the first embodiment;
Fig. 19 is a view showing a state where the securing unit is placed on an abdominal part and the camera to be installed in abdominal cavity is secured to the abdominal wall according to the first embodiment;
Fig. 20 is a sectional view showing the securing unit and the camera to be installed in abdominal cavity at the state of Fig. 19 according to the first embodiment;
Fig. 21 is a configuration view showing the entire endoscope system with the camera to be installed in abdominal cavity being secured to the abdominal wall according to the first embodiment;
Fig. 22 is a sectional view showing a configuration of a camera of a first modified example according to the first embodiment;
Fig. 23 is a perspective view showing a configuration of forceps according to the first embodiment;
Fig. 24 is a sectional view showing a configuration of a distal end portion of the forceps according to the first embodiment;
Fig. 25 is a perspective view showing a state where the camera is grasped by suction by the forceps according to the first embodiment;
Fig. 26 is a sectional view showing a configuration of a distal end portion of forceps of another form according to the first embodiment;
Fig. 27 is a sectional view showing a configuration of a housing of a camera and a distal end portion of forceps of another form according to the first embodiment;
Fig. 28 is a sectional view showing a configuration of a distal end portion of forceps of another form according to the first embodiment;
Fig. 29 is a perspective view showing a configuration of forceps and a pump unit for air-supply/suction of a second modified example according to the first embodiment;
Fig. 30 is a perspective view showing a housing of a camera and a distal end portion of forceps according to the first embodiment;
Fig. 31 is a sectional view showing a configuration of a housing of a camera and a distal end portion of forceps of another form according to the first embodiment;
Fig. 32 is a perspective view showing a configuration of a camera and a distal end portion of forceps of a third modified example according to the first embodiment;
Fig. 33 is a perspective view showing a configuration of a distal end portion of forceps of another form according to the first embodiment;
Fig. 34 is a sectional view showing a configuration of a distal end portion of the forceps of Fig. 33 according to the first embodiment;
Fig. 35 is a sectional view showing a configuration of a housing of a camera and a distal end portion of forceps of another form according to the first embodiment;
Fig. 36 is a sectional view showing a configuration of a grasp portion at a housing of a camera which is grasped by grasping forceps of a fourth modified example according to the first embodiment;
Fig. 37 is a sectional view showing a configuration of grasp portion at a housing of a camera and a distal end of forceps of another form according to the first embodiment;
Fig. 38 is a sectional view showing a configuration of an elastic member at a housing of a camera and a distal end portion of forceps having a needle portion of another form according to the first embodiment;
Fig. 39 is a sectional view showing a configuration of a snap-fit structure between a housing of a camera and a distal end portion of forceps of another form according to the first embodiment;
Fig. 40 is a perspective view showing a state where a camera of a fifth modified example is already received in a bag member of forceps according to the first embodiment;
Fig. 41 is a perspective view showing a state where a camera is being received in a bag member of forceps according to the first embodiment;
Fig. 42 is a perspective view showing a configuration of a camera in abdominal cavity according to a second embodiment of the present disclosure;
Fig. 43 is a longitudinal sectional view showing a configuration of the camera in abdominal cavity according to the second embodiment;
Fig. 44 is a transverse sectional view showing a configuration of the camera in abdominal cavity according to the second embodiment;
Fig. 45 is a longitudinal sectional view showing a configuration of a camera in abdominal cavity of a modified example according to the second embodiment;
Fig. 46 is a perspective view showing a configuration of a grasping unit with assist functions which is movable to a camera unit according to the second embodiment;
Fig. 47 is a perspective view showing a recess for grasping which is formed in a grasping unit with assist functions according to the second embodiment;
Fig. 48 is a plan view showing a configuration of a camera in abdominal cavity according to a third embodiment of the present disclosure;
Fig. 49 is a plan view showing the camera in abdominal cavity of Fig. 48 seen from the lateral direction thereof according to the third embodiment;
Fig. 50 is a plan view showing a camera in abdominal cavity of a modified example according to the third embodiment;
Fig. 51 is a plan view showing the camera in abdominal cavity of Fig. 50 seen from the lateral direction thereof according to the third embodiment;
Fig. 52 is a perspective view showing a configuration of a camera in abdominal cavity according to a fourth embodiment of the present disclosure;
Fig. 53 is a perspective view illustrating a configuration to cause a camera unit to rotate by an illumination unit mounting portion according to the fourth embodiment;
Fig. 54 is a view illustrating an operation to cause a camera unit to rotate by illumination unit mounting portion according to the fourth embodiment;
Fig. 55 is a view illustrating an operation to cause a camera unit to rotate by illumination unit mounting portion according to the fourth embodiment;
Fig. 56 is a perspective view showing a configuration of a camera in abdominal cavity according to a fifth embodiment of the present disclosure;
Fig. 57 is a plan view showing a configuration of illumination unit mounting portion according to the fifth embodiment;
Fig. 58 is a sectional view illustrating a configuration to cause a camera unit to rotate by illumination unit mounting portion according to the fifth embodiment;
Fig. 59 is a view illustrating a configuration of an illumination unit mounting portion that is rotatably operated about two axes according to the fifth embodiment;
Fig. 60 is a sectional view showing a configuration of a camera in abdominal cavity according to a sixth embodiment of the present disclosure;
Fig. 61 is a perspective view showing a configuration of a camera in abdominal cavity according to the sixth embodiment;
Fig. 62 is a plan view showing a configuration of a camera in abdominal cavity according to a seventh embodiment of the present disclosure;
Fig. 63 is a plan view showing the camera in abdominal cavity of Fig. 62 seen from the direction toward an observation window and an illumination window provided to the camera according to the seventh embodiment;
Fig. 64 is a view illustrating an operation to cause a camera in abdominal cavity to be introduced into an abdominal cavity using a treatment instrument according to the seventh embodiment;
Fig. 65 is a view illustrating an operation to cause a camera in abdominal cavity to be removed from the abdominal cavity using a treatment instrument to the outside of the body through a trocar according to the seventh embodiment; and
Fig. 66 is a plan view showing the camera in abdominal cavity of a modified example seen from the direction toward an observation window and an illumination window provided to the camera according to the seventh embodiment.

### DETAILED DESCRIPTION OF THE PREFERRED EMBODIMENTS

Now, several embodiments of the present disclosure will be explained below based on the drawings. In the following explanation, an example of a medical apparatus for laparoscopic surgery will be used.

### (First Embodiment)

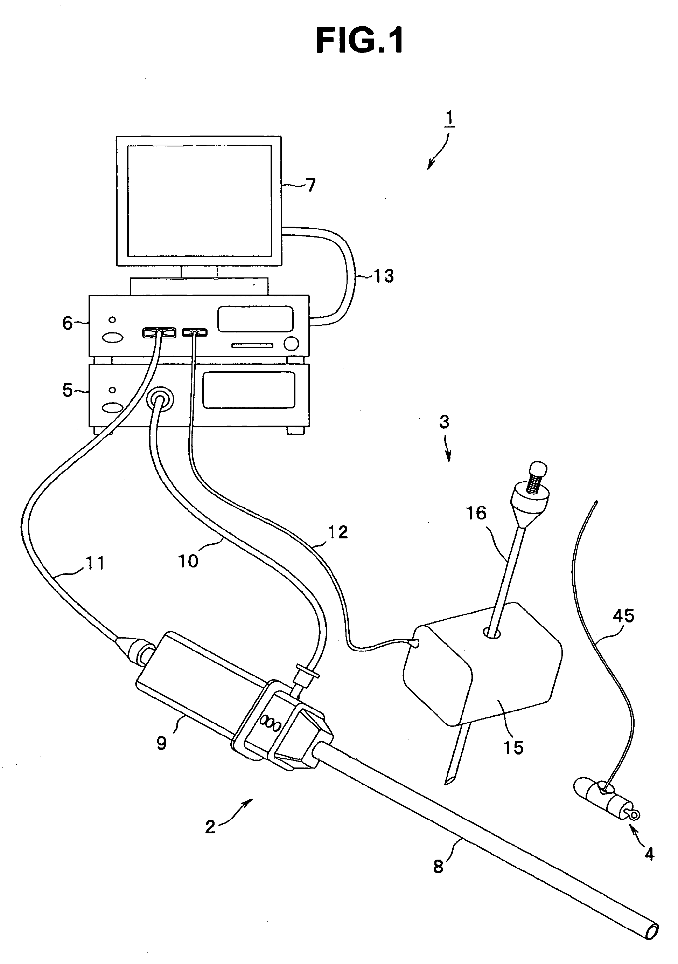

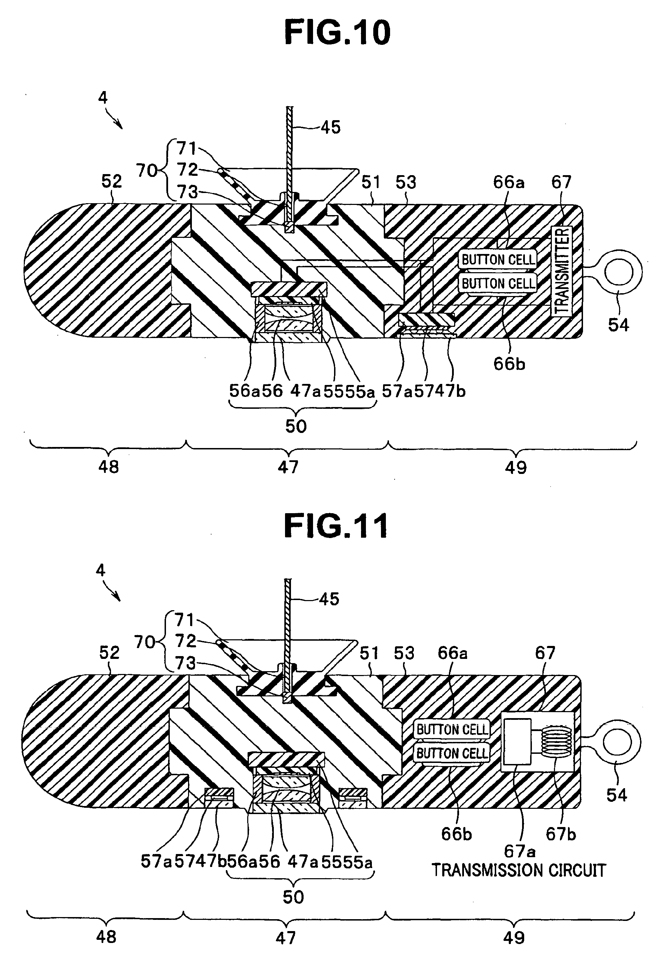

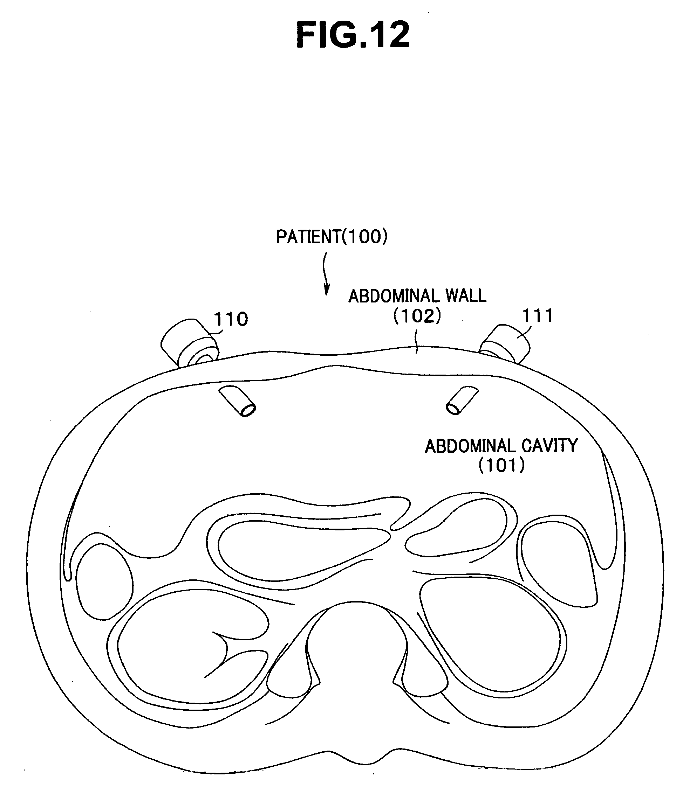

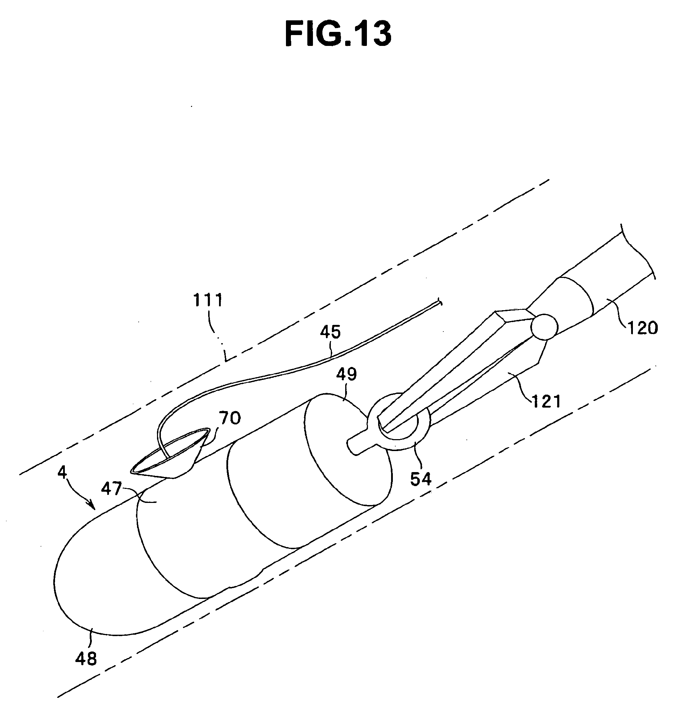

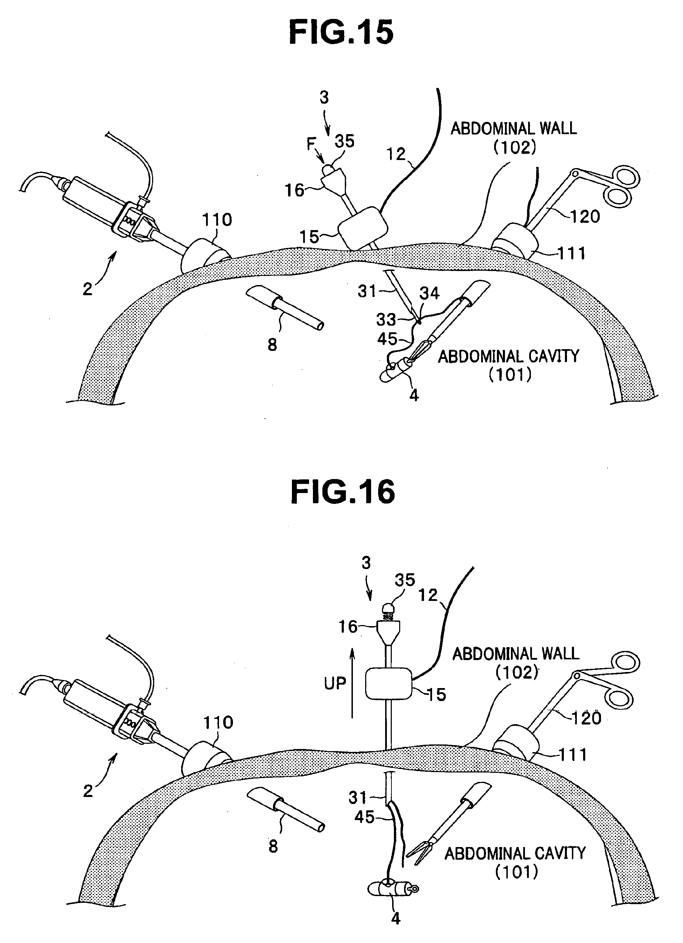

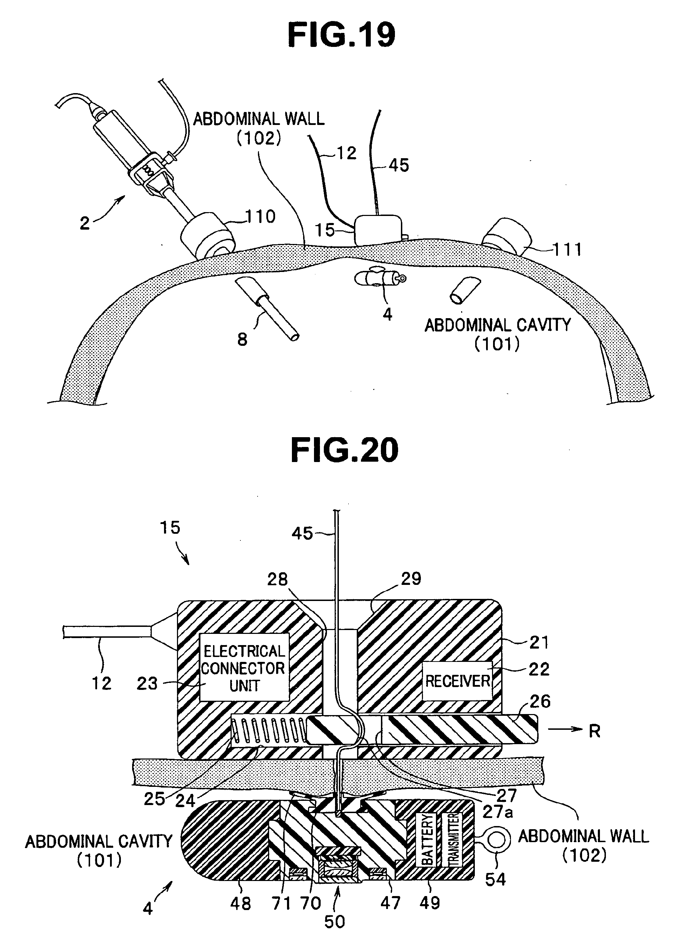

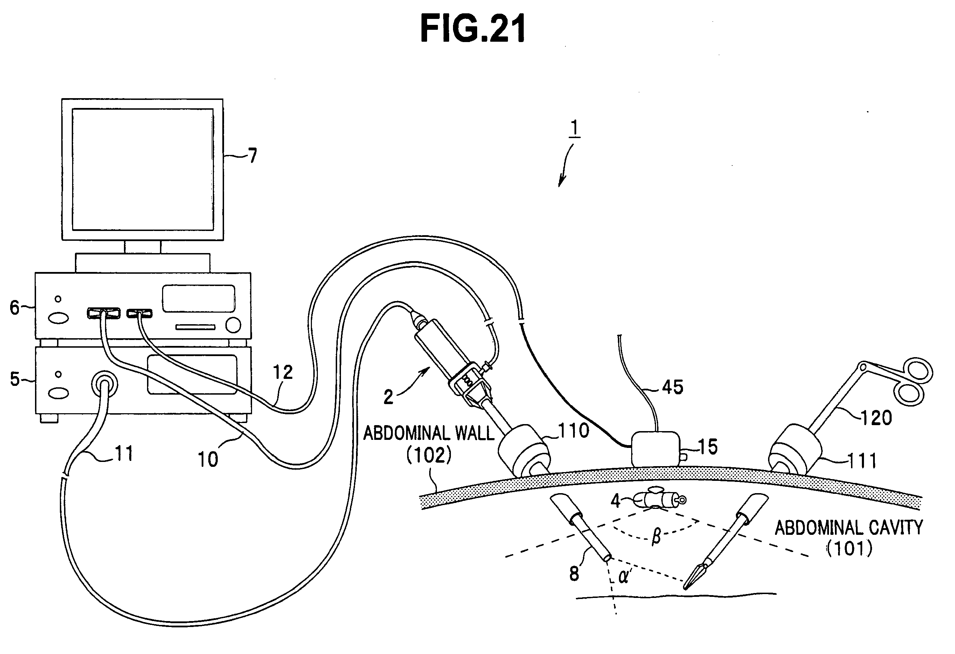

First, an endoscope system which is a medical apparatus used in laparoscopic surgery according to the present invention will be explained below. Figs. 1 to 21 show a first embodiment of the present invention: Fig. 1 is a view showing a configuration of an endoscope system that is a medical apparatus; Fig. 2 is a sectional view showing a configuration of an external apparatus; Fig. 3 is a top view showing an operation of a hook needle of the external apparatus; Fig. 4 is a sectional view showing a configuration of a camera to be installed in abdominal cavity; Fig. 5 is a sectional view taken along the V-V line of the Fig. 4; Fig. 6 is a sectional view showing a camera to be installed in abdominal cavity of a modified example; Fig. 7 is a sectional view showing a camera to be installed in abdominal cavity which is provided with various assist functions; Fig. 8 is a sectional view showing a camera to be installed in abdominal cavity having a button cell as a power source portion; Fig. 9 is a sectional view showing a camera to be installed in abdominal cavity having a hydrogen fuel cell as a power source portion; Fig. 10 is a sectional view showing a camera to be installed in abdominal cavity having an illumination portion arranged at a grasping unit with assist functions; Fig. 11 is a sectional view showing a camera to be installed in abdominal cavity to illustrate an internal configuration of a transmitter; Fig. 12 is a view showing a state where trocars are inserted through patient's abdominal wall; Fig. 13 is a perspective view showing a state where a grasp portion of a grasping unit with assist functions is grasped by grasping forceps; Fig. 14 is a view illustrating a procedure to introduce a camera to be installed in abdominal cavity into an abdominal cavity; Fig. 15 is a view showing a state where a hook needle is inserted through abdominal wall and a wire of a camera to be installed in abdominal cavity is engaged with the needle, and illustrating a procedure to introduce the camera to be installed in abdominal cavity into the abdominal cavity; Fig. 16 is a view showing a state where the hook needle engaged with the wire of the camera to be installed in abdominal cavity is pulled up, and illustrating a procedure to secure the camera to be installed in abdominal cavity to the abdominal wall; Fig. 17 is a view showing a state where the hook needle is pulled up and also a securing unit is pulled down along the hook needle, and illustrating a procedure to secure the camera to be installed in abdominal cavity to the abdominal wall; Fig. 18 is a sectional view for illustrating an operation of the external apparatus; Fig. 19 is a view showing a state where the securing unit is placed on an abdominal part and the camera to be installed in abdominal cavity is secured to the abdominal wall; Fig. 20 is a sectional view showing the securing unit and the camera to be installed in abdominal cavity at the state of Fig. 19; and Fig. 21 is a configuration view showing the entire endoscope system with the camera to be installed in abdominal cavity being secured to the abdominal wall.

As shown in Fig. 1, an endoscope system 1 of the present embodiment used in laparoscopic surgery is generally configured with a rigid endoscope 2 which is a first shooting apparatus, an external apparatus 3, an extremely small camera 4 to be installed in abdominal cavity which is a second shooting apparatus and also an image pickup apparatus (hereinafter, referred to as camera), a light source device 5, a camera control unit (hereinafter, simply referred to as CCU) 6 which is a signal processing device having an image process circuit incorporated therein, and a display unit 7 connected to the CCU 6 via a communication cable 13 configured to display an observation image.

The light source device 5 supplies illumination light to an illumination optical system provided in the rigid endoscope 2. The light source device 5 and the rigid endoscope 2 are removably connected to each other via a light source cable 10.

The rigid endoscope 2 is mainly configured with a rigid insertion portion 8, and an operation portion 9 connected to a proximal end of the insertion portion 8. The insertion portion 8 of the rigid endoscope 2 includes an image guide and a light guide bundle inserted therethrough, and has a shooting optical system configured to focus an image of a photographic subject onto a camera for rigid endoscope (which will be explained later) via the image guide, and an illumination optical system configured to emit an illumination light from the light guide bundle to a photographic subject, at the surface of a distal end of the insertion portion 8.

The operation portion 9 of the rigid endoscope 2 has a camera head incorporated therein (not shown) in which a solid-state image pickup element such as CCD and CMOS is arranged. When an illumination light supplied from light source device 5 via the light source cable 10 to the rigid endoscope 2 illuminates a region to be observed, an optical image of the region is picked up by the camera head in the operation portion 9 through the image guide of the insertion portion 8. The camera for rigid endoscope photoelectrically converts the picked up optical image into an image pick up signal, which is transmitted to the CCU 6 via an image pick up cable 11. The rigid endoscope 2 of the present embodiment is provided with an image pickup optical system to set an angle of view α (see Fig. 21) for shooting to be within a range of 70 degrees to 75 degrees for example.

The CCU 6 generates a video signal from the transmitted image signal and outputs the video signal to the display unit 7. The display unit 7 is a liquid crystal display for example, which displays a normal observation image picked up by the rigid endoscope 2 and a wide-angle observation image picked up by the camera 4 in two screens simultaneously, or individually by switching, upon receiving the video signal outputted from the CCU 6. The CCU 6 is removably connected to a securing unit 15 of the external apparatus 3 via an electrical cable 12 which will be explained later.

Next, the external apparatus 3 will be explained below in detail with reference to Figs. 2 and 3.

The external apparatus 3 is configured with, as shown in Figs. 2 and 3, the securing unit 15 configured to draw the camera 4 into a body cavity for securing, and a hook needle 16 which is a puncture needle configured to be engage with the camera 4 to pull up the camera 4.

The securing unit 15 has a receiver 22 and an electrical connector unit 23 electrically connected to the receiver 22 incorporated in a housing 21 which is formed of a non-magnetic material. The electrical connector unit 23 is connected to an electrical cable 12 which is connected to the CCU 6. The securing unit 15 transmits electric power from the CCU 6 and signals from the receiver 22 to the CCU 6 via the electrical cable 12.

The housing 21 has a slide hole 24 formed in the lateral direction from one side surface thereof. The slide hole 24 includes a wire securing lever 26 inserted therethrough, and the wire securing lever 26 constitutes a securing portion having a bias spring 25 fixed at one end surface thereof and formed of a non-magnetic member. The wire securing lever 26 has a generally rectangular shape, and is slidably disposed in the direction toward the inside of the housing 21 along the slide hole 24. The wire securing lever 26 includes an opening 27 at the middle thereof that has a convex circular surface 27a on the bias spring 25 side thereof.

The housing 21 has a wire insertion portion 28 formed vertically therethrough. The wire insertion portion 28 flares upwardly with a conically tapered surface 29 at the top portion thereof, which forms an upper aperture of the housing 21.

In the securing unit 15 configured as described above, at a slide position where the opening 27 of the wire securing lever 26 is aligned with the wire insertion portion 28 when the wire securing lever 26 is inserted into the housing 21, a vertical hole is formed through the housing 21, through which the hook needle 16 is slidably inserted.

The hook needle 16 of the external apparatus 3 is configured to have a cylindrical puncture needle tube 31, a needle head 32 integrally connected to the upper portion of the puncture needle tube 31, a puncture rod 33 having a hook portion 34 at the distal end thereof that is configured to be slidably inserted through the puncture needle tube 31, a hook head 35 integrally connected to the upper portion of the puncture rod 33, and a spring 36 interposed between the hook head 35 and the needle head 32.

The puncture needle tube 31 is an elongated metal tube member that has a diameter of about 3 mm and includes a distal end, the end being cut at an oblique angle and formed into a sharp needle shape. The needle head 32 has a diameter larger than that of the puncture needle tube 31, and is formed into a conical shape on the distal end side thereof to be integrally formed with the puncture needle tube 31. The needle head 32 is configured to contact the tapered surface 29 formed at the upper portion of the housing 21 so that the hook needle 16 does not slip down through the housing 21.

The puncture rod 33 is an elongated metal bar, and has the hook head 35 integrally formed at the upper portion that is biased by the spring 36 in the direction away from the needle head 32. This configuration allows the hook portion 34 formed at the distal end of the puncture rod 33 to be received inside of the puncture needle tube 31.

When the hook head 35 of the hook needle 16 is pressed into the puncture needle tube 31 against the bias of the spring 36 by a user (the arrow F of Fig. 3), the hook portion 34 at the distal end of the puncture rod 33 is projected out from the distal end of the puncture needle tube 31.

The hook needle 16 configured as described above is, when threaded through the wire insertion portion 28 of the housing 21 and the opening 27 of the wire securing lever 26, fixed by insertion in the housing 21 due to the pressure in the direction to the outside of the housing 21 which is caused by the bias of the bias spring 25 of the wire securing lever 26. In other words, the hook needle 16 is fixed by insertion in the housing 21 because the outer peripheral surface of the puncture needle tube 31 is pressed by the circular surface 27a formed on one side surface of the opening 27 of the wire securing lever 26 and contacts the inner surface of the wire insertion portion 28.

Next, the camera 4 will be explained below in detail with reference to Figs. 4 and 5.

The camera 4 is, as shown in Fig. 4, mainly configured with a camera unit 47 which is an image pickup portion, a balancing member 48 fitted into one side of the camera unit 47 in a row arrangement, and a grasping unit 49 with assist functions incorporated therein which is fitted into the other side of the camera unit 47 in a row arrangement. The camera 4 is a capsule-type image pickup unit having an outer surface of bullet-shape on the balancing member 48 side.

The camera unit 47 is provided with an image pickup unit 50 and a plurality of (two in the present embodiment) small illumination portions 57 of low power consumption in the generally cylindrical camera housing 51, the illumination portions 57 including an LED or organic ELD as a light source of illumination light.

The image pickup unit 50 is mainly configured with a solid-state image pickup device 55 such as CCD and CMOS, an image pickup device drive circuit portion 55a configured to drive-control the solid-state image pickup device 55 and photoelectrically convert an image pickup light incident to the solid-state image pickup device 55, object lenses 56 configured to focus the image pickup light onto the solid-state image pickup device 55, and a lens support frame 56a configured to support the object lenses 56.

The illumination portion 57 is mounted on an illumination drive circuit portion 57a to be drive-controlled. The camera housing 51 is provided with transparent cover members 47a and 47b covering the image pickup unit 50 and each of the illumination portions 57 in a water-tight manner.

The image pickup unit 50 in the camera unit 47 of the present embodiment is provided with an image pickup optical system for picking up a wide field of view to set an angle of view β (see Fig. 21) for shooting to be within a range of 90 degrees or more for example.

The camera housing 51 of the camera unit 47 is fitted with an abdominal wall securing portion 70 at the outer peripheral portion thereof on the side opposite to the image pickup direction of the image pickup unit 50 (the direction along the y-axis of Fig. 4). The abdominal wall securing portion 70 is formed with an elastic member of a material such as silicon rubber for example, and is configured with a suction cup 71 on the camera 4 side thereof. The abdominal wall securing portion 70 also has a through hole 72 formed centrally thereof. The abdominal wall securing portion 70 is configured to be removable from the camera housing 51 for replacement when the abdominal wall securing portion 70 itself is degraded.

Into the through hole 72 of the abdominal wall securing portion 70, a wire 45 having a predetermined length for lifting is inserted therethrough, and the wire has a coupling portion 73 connected to one end of the wire 45 by crimping. The coupling portion 73 is fitted in the camera housing 51 for fixing. That is, the wire 45 is provided to be extended out from the center of the suction cup 71. The suction cup 71 formed of an elastic member closely contacts with a body wall (abdominal wall) with the end thereof being under stretched deformation when the wire 45 is pulled up at a certain amount of tension or more. The wire 45 may be a thread such as surgical suture, or a metal stranded wire.

The balancing member 48 is a member designed to have a weight generally the same as that of the grasping unit 49 with assist functions incorporated therein so as to stable the balance of the camera 4 for securing when the camera 4 is secured to a body wall (abdominal wall) using the abdominal wall securing portion 70 of the camera unit 47.

The balancing member 48 includes a body portion 52 that is formed of a synthetic resin and has the same outer shape as that of the camera housing 51 of the camera unit 47, so that the balancing member 48 is fitted with the side of the camera housing 51 that has the shape. The balancing member 48 is configured to be removable from the camera housing 51 for replacement with another balancing member having a different weight to stable the balance of the camera 4 depending on the weight of the grasping unit 49 with assist functions incorporated therein.

The grasping unit 49 with assist functions incorporated therein includes a transmitter 67 and a battery 66 in a housing 53 formed of a synthetic resin: the transmitter 67 is an assistance apparatus configured to wirelessly transmit image pickup signals from the image pickup unit 50 in the camera unit 47 to the outside, and the battery 66 provides a power source portion which is an assistance apparatus configured to supply power to the image pickup unit 50, each of the illumination portion 57, and each of the illumination drive circuit portion 57a of the illumination unit. The image signals photoelectrically converted by the image pickup unit 50 are wirelessly transmitted from the transmitter 67 to the receiver 22 disposed in the housing 21 of the external apparatus 3.

The assistance apparatuses including the battery 66 and the transmitter 67 are arranged in parallel in the housing 53 of the grasping unit 49 with assist functions in the direction (the direction along the x-axis of Fig. 4) generally orthogonal to the viewing direction of the image pickup unit 50 in the camera unit 47 (the direction for shooting) (the direction along the y-axis of Fig. 4).

The housing 53 of the grasping unit 49 with assist functions has the same outer shape as that of the camera housing 51 of the camera unit 47 to be fitted with the other side of the camera housing 51 that has the shape. The housing 53 is provided with a ring-shaped grasp portion 54 which projects from the center of the end surface of one end of the camera 4 in the longitudinal direction of the camera 4. The grasp portion 54 is sandwiched between treatment portions of grasping forceps when introduced in a patient's body (an abdominal cavity 101).

The grasping unit 49 with assist functions is configured to be removable from the camera housing 51 for replacement when the inside battery 66 runs short of power or the grasp portion 54 itself is degraded. To be removable from the housing 53, the grasp portion 54 may be configured to be solely removed for replacement. This configuration allows the battery 66 that can be charged through an external terminal to be included in the grasping unit 49 with assist functions.

The camera 4 including the abdominal wall securing portion 70 has, as shown in Fig. 5, an outer shape in the lateral direction which is designed to be insertable into a trocar 111 (see Figs. 13 to 17) which will be explained later. In other words, the camera 4 is configured to have an outer shape that can be inserted into the trocar 111 along the longitudinal direction thereof so as to be insertable into a patient's body (the abdominal cavity 101) through the trocar 111 when the grasp portion 54 of the grasping unit 49 with assist functions is sandwiched between the treatment portion of grasping forceps.

Moreover, the camera 4 of the present embodiment is, as described above, configured to facilitate the insertion operation into the trocar 111 with the grasp portion 54 sandwiched between the treatment portion of grasping forceps because the grasp portion 54 is centrally provided on the end surface of the grasping unit 49 with assist functions which constitutes one side end portion of the camera 4 in the longitudinal direction thereof.

Furthermore, the camera 4 is configured not to have an outer shape which increases in the lateral direction (the direction along the y-axis of Fig. 4), that is, configured to have a low profile, by arranging the battery 66 and the transmitter 67 in parallel in the grasping unit 49 with assist functions in the direction generally orthogonal to the viewing direction (the direction for shooting) of the image pickup unit 50. This configuration enables a shooting of a subject to be examined within a wider range when the camera 4 is disposed in a patient's body (the abdominal cavity 101), and also prevents interference of the camera 4 with other medical device and medical tool that are similarly introduced in the body.

The image pickup unit 50 may be, as shown in Fig. 6, arranged to have a viewing direction (the direction for shooting) which forms a predetermined angle θ relative to the y-axis in the longitudinal direction of the camera 4. This configuration prevents interference of the camera 4 with other medical device and medical treatment instrument that are similarly introduced in the body, depending on the position where the camera 4 is placed in body (the abdominal cavity 101).

The grasping unit 49 with assist functions of the camera 4 may, as shown in Fig. 7, include various assistance apparatuses 58 and 59 in addition to the battery 66 and the transmitter 67. Similar to the battery 66 and the transmitter 67, the assistance apparatuses 58 and 59 are arranged in parallel in the direction generally orthogonal to the viewing direction (the direction for shooting) of the image pickup unit 50, so that the camera 4 is configured not to have an outer shape which increases in the lateral direction and realize a low profile.

Specifically, the various assistance apparatuses 58 and 59 may include: a temperature sensor for sensing a temperature in body (the abdominal cavity 101); a humidity sensor for sensing a humidity in body (the abdominal cavity 101); a pressure sensor for sensing a pressure in body (the abdominal cavity 101); a tilt sensor for sensing a tilt of the camera 4 using an acceleration sensor; and a position detecting sensor such as GPS for detecting a position of the camera 4 in body (the abdominal cavity 101), for example.

The power source portion which is an assistance apparatus incorporated in the grasping unit 49 with assist functions of the camera 4 may be, as shown in Fig. 8, button cells 66a and 66b, instead of the battery 66. Fig. 8 shows an example having the two button cells 66a and 66b that are disposed onto each other in series in the y-axis of Fig. 8. In other words, the two button cells 66a and 66b are transversely superimposed on each other along the x-axis of Fig. 8 with the sides thereof having a smaller thickness being vertically arranged, so that the camera 4 is configured not to have an outer shape which increases in the direction along the y-axis at the grasping unit 49 with assist functions. This prevents the camera 4 from having an outer shape which increases in the lateral direction, that is the direction along the y-axis of Fig. 8, (and realize a low profile), and enables a shooting of a subject to be examined within a wider range, and also prevents interference of the camera 4 with other medical device and medical tool that are similarly introduced in the body (the abdominal cavity 101).

Furthermore, the power source portion which is an assistance apparatus incorporated in the grasping unit 49 with assist functions of the camera 4 may be, as shown in Fig. 9, a hydrogen fuel cell that includes a power generator unit 66c and a hydrogen tank 66d, instead of the battery 66. The power generator unit 66c and the hydrogen tank 66d are also arranged with the transmitter 67 in parallel in the direction generally orthogonal to the viewing direction (the direction for shooting) of the image pickup unit 50 to be incorporated in the grasping unit 49 with assist functions, so that the camera 4 is configured not to have an outer shape which increases in the lateral direction (the direction along the y-axis of Fig. 9) and realize a low profile. This allows the camera 4 to be positioned far away from a subject to be examined in body (the abdominal cavity 101) for a shooting within a wider range of observation, and also prevents interference of the camera 4 with other medical device and medical tool that are similarly introduced in the body.

The illumination portion 57 including an LED, an organic ELD and the like and the illumination drive circuit portion 57a that constitutes an illumination unit may be, as shown in Fig. 10, incorporated in the grasping unit 49 with assist functions. In the case, the grasping unit 49 with assist functions includes the illumination unit configured with each of the illumination portion 57 and the illumination drive circuit portion 57a, and the two button cells 66a and 66b here, and the transmitter 67 that are arranged in parallel in the direction (the direction along the x-axis of Fig. 9) generally orthogonal to the viewing direction (the direction for shooting) of the image pickup unit 50, which prevents the camera 4 from having an outer shape which increases in the lateral direction (the direction along the y-axis of Fig. 9), and allows the camera 4 to have a low profile.

The transmitter 67 is, as shown in Fig. 11, configured to have a transmission circuit 67a and an antenna portion 67b, and the transmission circuit 67a and the antenna portion 67b are arranged in parallel in the direction (the direction along the x-axis of Fig. 11) generally orthogonal to the viewing direction (the direction for shooting) of the image pickup unit 50, which prevents the camera 4 from having an outer shape which increases in the lateral direction (the direction along the y-axis of Fig. 11), and allows the camera 4 to have a low profile.

The endoscope system 1 of the present embodiment configured as described above is used in laparoscopic surgery, and is used in treatment in abdominal cavity that is one of a patient's body cavities.

Now, a procedure and operation to place the endoscope system 1 of the present embodiment in abdominal cavity that is a patient's body cavity for laparoscopic surgery will be explained below in detail with reference to Figs. 12 to 21.

First, a surgeon makes two small incisions at abdominal wall 102 of a patient 100 with a knife or the like, and as shown in Fig. 12, inserts trocars 110 and 111 into the incisions. At this point of time, the surgeon makes another incision at the abdominal wall 102 at a position separated from the trocar 110 which is used to insert the rigid endoscope 2 into the abdominal cavity 101 by a predetermined distance in the present embodiment, and inserts the trocar 111 through the incision into the abdominal cavity 101 to insert a treatment instrument 120 such as grasping forceps into the abdominal cavity 101.

The surgeon also, as shown in Figs. 2 and 3, inserts the puncture needle tube 31 of the hook needle 16 into the wire insertion portion 28 formed in the securing unit 15 of the external apparatus 3. The surgeon presses the wire securing lever 26 into the housing 21 so that the puncture needle tube 31 passes through the opening 27 in the wire securing lever 26 and thereby the securing unit 15.

The surgeon causes the puncture needle tube 31 to be sufficiently projected out from the lower surface of the securing unit 15 (see Figs. 2 and 3) with the securing unit 15 being placed at a position on the needle head 32 side that is the proximal end side of the puncture needle tube 31. In the state, the bias of the bias spring 25 applied to the wire securing lever 26 causes the circular surface 27a which is a wall surface of the opening 27 of the wire securing lever 26 to contact and hold the puncture needle tube 31, thereby the securing unit 15 is configured not to be slipped down along the puncture needle tube 31.

Next, the surgeon inserts the insertion portion 8 of the rigid endoscope 2 into the abdominal cavity 101 through the trocar 110 (see Fig. 14). Then, the surgeon inserts the camera 4 into the abdominal cavity 101 through the trocar 111, while grasping the camera 4 using the treatment instrument 120 such as grasping forceps. In the insertion, the surgeon preferably checks the images picked up by the rigid endoscope 2 while inserting the camera 4 into the abdominal cavity 101.

When the camera 4 is inserted into the abdominal cavity 101 through the trocar 111, as shown in Fig. 13, the grasp portion 54 of the grasping unit 49 with assist functions is grasped, being sandwiched by a treatment portion 121 of the treatment instrument 120 such as grasping forceps. The grasp portion 54 is configured so that the treatment instrument 120 easily grasps the camera 4 along the longitudinal direction of the camera 4 in a well balanced condition. This facilitates an insertion of the camera 4 by a surgeon through the trocar 111 into abdominal cavity: that is, the camera 4 can be easily introduced into the abdominal cavity 101 without being trapped by the trocar 111 by a surgeon.

Next, the surgeon punctures the abdominal wall 102 with the puncture needle tube 31 of the hook needle 16 inserted to and held by the securing unit 15 of the external apparatus 3, as shown in Figs. 14 and 15, while checking the images by the rigid endoscope 2. Then, as shown in Fig. 15, the surgeon presses the hook head 35 in the direction shown by the arrow F of Fig. 15 to cause the puncture rod 33 to be projected out from the puncture needle tube 31. With the puncture rod 33 being projected out, the surgeon hooks the hook portion 34 of the puncture rod 33 onto the wire 45 of the camera 4 while viewing the images by the rigid endoscope 2.

When the hook portion 34 is hooked onto the wire 45, the surgeon releases the pressing of the hook head 35 of the puncture rod 33, which causes the puncture rod 33 to be drawn back into the puncture needle tube 31 with the wire 45 being hooked by the hook portion 34.

After that, as shown in Fig. 16, the surgeon retracts the puncture needle tube 31 of the hook needle 16 out of the abdominal cavity 101 to the outside of the body (in the upward direction of Fig. 16) with hook portion 34 of the puncture rod 33 having the wire 45 engaged thereto. Then, as shown in Fig. 17, the surgeon retracts the puncture needle tube 31 of the hook needle 16 out of the abdominal cavity 101 and also moves the securing unit 15 relative to the puncture needle tube 31 in the direction toward the abdominal part of the patient 100 (in the downward direction of Fig. 16) to draw the puncture needle tube 31 until the wire 45 passes through the wire insertion portion 28 of the securing unit 15.

In the drawing, the surgeon presses the wire securing lever 26 of the securing unit 15 into the housing 21 (in the direction shown by the arrow P of Fig. 18), which facilitates the relative sliding of the securing unit 15 to the puncture needle tube 31 of the hook needle 16. Then, when the wire 45 passes through the wire insertion portion 28 of the securing unit 15, as shown in Fig. 18, the surgeon draws the wire 45 itself (in the upward direction of Fig. 18), and also moves the securing unit 15 in the direction toward the abdominal part of the patient 100 (in the downward direction of Fig. 18) relative to the wire 45 this time.

That is, the surgeon can easily relatively slide the securing unit 15 to the puncture needle tube 31 of the hook needle 16 and the wire 45 of the camera 4 while the wire securing lever 26 of the securing unit 15 is pressed into the housing 21.

As shown in Fig. 19, the surgeon draws the wire 45 of the camera 4 until the abdominal wall 102 is sandwiched between the securing unit 15 and the camera 4 with the securing unit 15 being rested on the abdominal part of the patient 100. In the drawing, after confirming the attachment of the suction cup 71 of the camera 4 to the inner surface of the abdominal wall 102 by checking the image obtained by the rigid endoscope 2, as shown in Fig. 20, the surgeon releases the pressing of the wire securing lever 26 of the securing unit 15.

Then, the wire securing lever 26 of the securing unit 15 moves in the direction shown by the arrow R shown in Fig. 20 due to the bias of the bias spring 25, which causes the opening 27 to be offset from the wire insertion portion 28 of the housing 21, resulting in that the wire 45 through the opening 27 and the wire insertion portion 28 is sandwiched and secured in the housing 21. Because the elastic suction cup 71 deforms, at least a certain amount of tension is constantly applied to the wire 45 between the wire securing lever 26 and the suction cup 71. Due to the tension constantly applied to the wire 45, the securing unit 15 and the camera 4 are held and secured in the state of sandwiching the abdominal wall 102 therebetween.

In the way, as shown in Fig. 21, the camera 4 is placed in the abdominal cavity 101 of the patient 100 in a quite stable condition, and then laparoscopic surgery is started using the endoscope system 1 of the present embodiment. To the trocar 110, for example, a pneumoperitoneum tube (not shown) is connected at one end thereof, and into the abdominal cavity 101, carbon dioxide gas is injected for example as a gas for pneumoperitoneum in order to maintain the observation field of view of the rigid endoscope 2 and to maintain the area for the operation of devices for surgery. Then, the surgeon inserts the rigid endoscope 2 through the trocar 110, and treatment instrument 120 through the trocar 111 to start laparoscopic surgery, with the camera 4 being attached and placed to the abdominal wall 102 in the abdominal cavity 101.

After laparoscopic surgery is completed, the surgeon extracts the securing unit 15 from the wire 45 while pressing the wire securing lever 26 of the securing unit 15 into the housing 21. Then, the surgeon grasps the camera 4 in the abdominal cavity 101 with the treatment instrument 120 such as grasping forceps, and takes the camera 4 out of the abdominal cavity 101 through the trocar 111.

According to the endoscope system 1 of the above described embodiment, body tissues in body cavity that is the abdominal cavity 101 in the above embodiment can be observed from multiple view points within a wide angle, which facilitates the determination of the entire incision line for surgery of a large organ or for incision of large intestine, for example. The endoscope system 1 also realizes a minimally invasive surgery without increasing any burden to patient when the camera 4 is placed in the abdominal cavity 101 in addition to the rigid endoscope 2 for magnified observation. As a result, the use of an endoscope system 1 according to the present invention facilitates the treatment in laparoscopic surgery.

Moreover, because the camera 4 has a small and low-profile configuration by incorporating various assistance apparatuses in the grasping unit 49 with assist functions in parallel in the direction which is generally orthogonal to the viewing direction (the direction for shooting) of the image pickup unit 50, the camera 4 in body (the abdominal cavity 101) achieves an image pickup within a wider range of observation at a position far away from a subject to be examined, and also prevents interference of itself with other medical device and medical tool that are similarly introduced in the body.

### (First Modified Example)

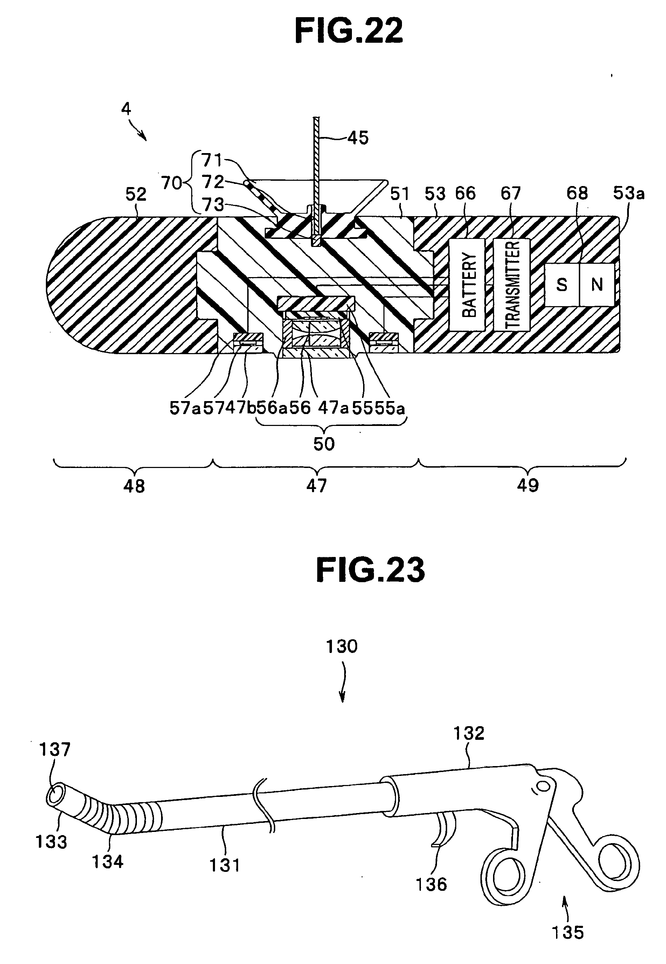

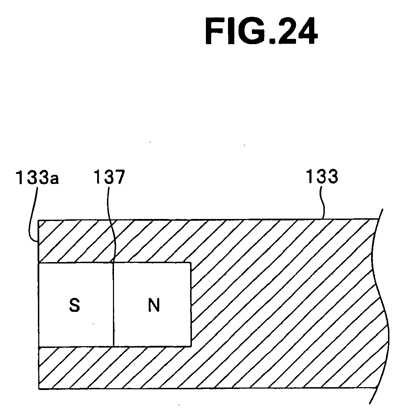

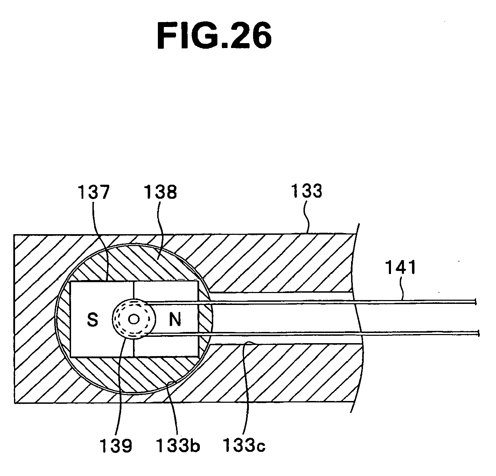

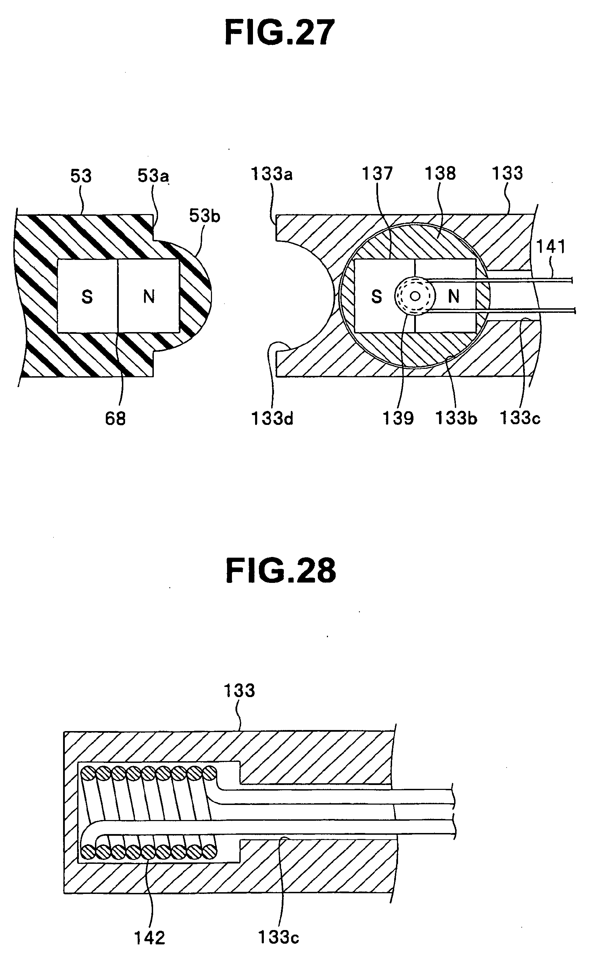

Now, a first modified example of the present embodiment will be explained below with reference to Figs. 22 to 28. Figs. 22 to 28 show the first modified example: Fig. 22 is a sectional view showing a configuration of a camera; Fig. 23 is a perspective view showing a configuration of forceps; Fig. 24 is a sectional view showing a configuration of a distal end portion of the forceps; Fig. 25 is a perspective view showing a state where the camera is grasped by suction by the forceps; Fig. 26 is a sectional view showing a configuration of a distal end portion of forceps of another form; Fig. 27 is a sectional view showing a configuration of a housing of a camera and a distal end portion of forceps of another form; and Fig. 28 is a sectional view showing a configuration of a distal end portion of forceps of another form.

As shown in Fig. 22, the housing 53 of the grasping unit 49 with assist functions of the camera 4 does not have the grasp portion 54, but has a permanent magnet 68 incorporated therein near an end surface 53a in the direction generally orthogonal to the viewing direction (the direction for shooting) of the image pickup unit 50 in the camera unit 47.

In the present modified example also, special forceps 130 shown in Fig. 23 is used to introduce the camera 4 into the abdominal cavity 101 through the trocar 111.

The forceps 130 includes an insertion portion 131 and an operation portion 132 connected to the insertion portion 131. The insertion portion 131 has a distal end portion 133 and a bending portion 134. The bending portion 134 is configured to be bent by two circular hand grips 135 of the operation portion 132. The operation portion 132 also includes an operation lever 136 for other functions.

The forceps 130 includes a permanent magnet 137 having polarities (S/N) at the distal end portion 133, as shown in Fig. 24, to cause attractive force between the permanent magnet 137 and the permanent magnet 68 in the camera 4. The permanent magnet 137 is arranged to be exposed while being flush with the distal end surface 133a of the distal end portion 133.

As shown in Fig. 25, in introducing the camera 4 into the abdominal cavity 101, the forceps 130 is attached to the camera 4 so that the distal end surface 133a of the distal end portion 133 is in surface contact with the end surface 53a of the grasp portion 54 of the camera 4. In the state, due to the attractive force between the permanent magnet 68 of the camera 4 and the permanent magnet 137 of the forceps 130, the camera 4 is held by the forceps 130. The distal end portion 133 of the forceps 130 is set to have an outer diameter that is equal to or less than that of the grasping unit 49 with assist functions of the camera 4.

As a result, the camera 4 of the present modified example can be attached to the forceps 130 substantially coaxially with the distal end portion 133, to be inserted into the abdominal cavity 101 without being trapped by the trocar 111. After introduced in the abdominal cavity 101, due to a predetermined stress larger than the attractive force of the permanent magnets 68 and 137, the camera 4 is easily removed from the forceps 130.

The distal end portion 133 of the forceps 130 may be configured to include the permanent magnet 137 therein in a rotatable manner, so that the direction of the polarities (S/N) of the permanent magnet 137 can be changed, as shown in Fig. 26.

Specifically, the distal end portion 133 is provided with a disc-shaped rotary member 138 in which the permanent magnet 137 is embedded. The rotary member 138 has a pulley 139 therein. An operation wire 141 is wound to turn around the pulley 139 so as to rotate the rotary member 138 together with the permanent magnet 137.

The distal end portion 133 has a hold hole 133b that has a circular cross section and rotatably holds the rotary member 138, and a through hole portion 133c which is in communication with the hold hole 133b and provides a threading path for the operation wire 141. The operation wire 141 is, not shown but, threaded through the insertion portion 131 of the forceps 130 to be drawn and released in accordance with the operation of the operation lever 136 of the operation portion 132. That is, the rotation of the rotary member 138 is caused by the operation lever 136 of the operation portion 132, which changes the direction of the polarities (S/N) of the permanent magnet 137.

In the above configuration, the forceps 130 is able to make a switch between attractive force and repulsive force to the permanent magnet 68 of the camera 4 by changing the direction of the polarities (S/N) of the permanent magnet 137 in accordance with the operation of the operation lever 136 of the operation portion 132, thereby the forceps 130 is able to make a switch between the operation to hold the camera 4 to introduce it into the abdominal cavity 101 and the operation to separate the camera 4 therefrom in the abdominal cavity 101 or outside of the body.

Alternatively, as shown in Fig. 27, the end surface 53a of the housing 53 of the grasping unit 49 with assist functions in the camera 4 may have a semi-spherical projection 53b, and a semi-spherical recess 133d may be formed in the distal end surface 133a of the distal end portion 133 of the forceps 130 to engage the projection 53b, for example.

The above configuration ensures the coaxially engaged attachment of the distal end portion 133 of the forceps 130 to the camera 4, which facilitates the alignment of the attachment between the camera 4 and the distal end portion 133, and also enhances the gripping force of the forceps 130 to the camera 4. Thus, in securing the camera 4 to the abdominal wall 102, even the forceps 130 performs a bending operation of the bending portion 134, the camera 4 is not easily removed from the distal end portion 133, resulting in the improved operability of the camera 4 in the abdominal cavity 101.

Instead of the permanent magnet 137 in the distal end portion 133, an electromagnet in a form of a coil 142 may be used, as shown in Fig. 28. The coil 142 that provides an electromagnet is configured to generate a magnetic force upon an application of a current by a battery (not shown) in the operation portion 132 or an external power source.

The operation lever 136 of the operation portion 132 may be configured to apply a current, so that a change in the direction of the current application causes the polarities (S/N) generated by the coil 142 to be changed, or the operation lever 136 may be simply configured to turn on/off the current application.

### (Second Modified Example)

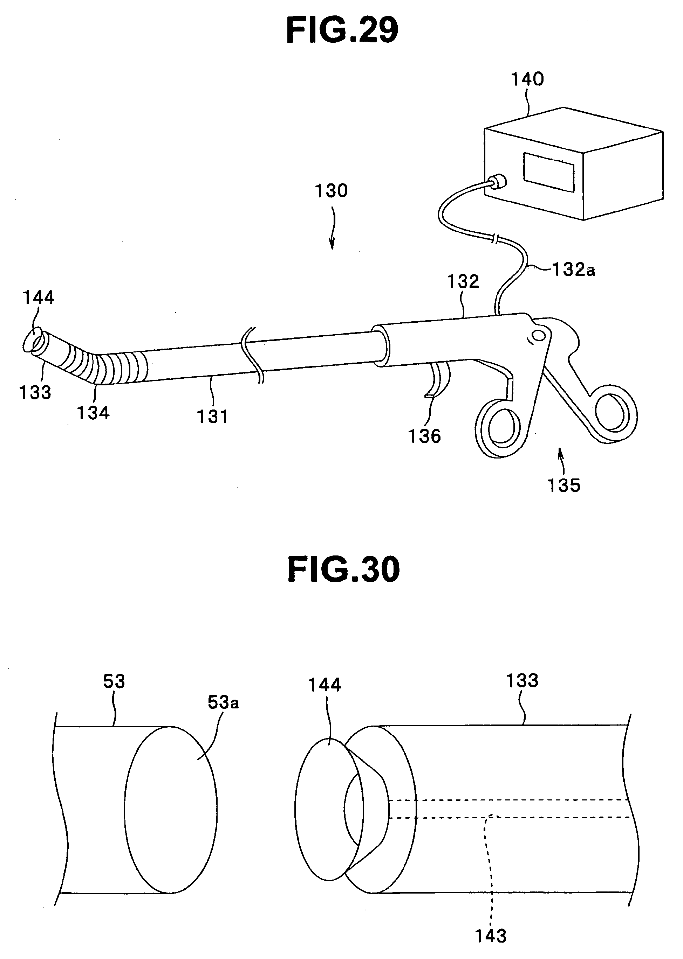

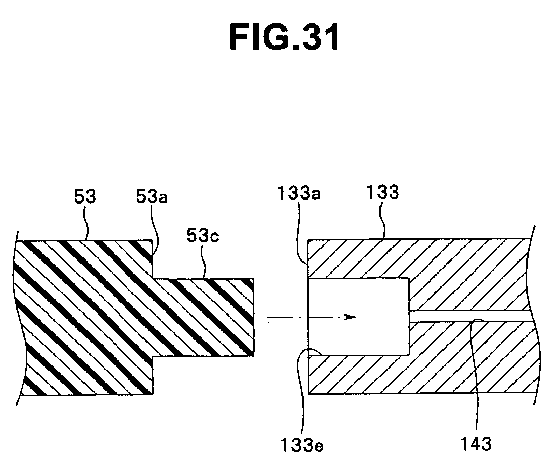

Next, a second modified example of the present embodiment will be explained below with reference to Figs. 29 to 31. Figs. 29 to 31 show the second modified example: Fig. 29 is a perspective view showing a configuration of forceps and a pump unit for air-supply/suction; Fig. 30 is a perspective view showing a housing of a camera and a distal end portion of forceps; and Fig. 31 is a sectional view showing a configuration of a housing of a camera and a distal end portion of forceps of another form.

Forceps 130 of the present modified example have a suction cup portion 144 at the distal end surface 133a of the distal end portion 133, as shown in Figs. 29 and 30. The suction cup portion 144 has a hole portion formed in the center thereof which is in communication with an air-supply/suction channel 143 through the insertion portion 133 of the forceps 130.

The forceps 130 also has an air-supply/suction tube 132a extending from the operation portion 132, with an electrical wire (not shown) being arranged in the air-supply/suction tube 132a. The air-supply/suction tube 132a is connected to an air-supply/suction pump unit 140. The air-supply/suction pump unit 140 is operated by the operation lever 136 of the operation portion 132 of the forceps 130 to supply air, suck air, and stop the supply/suction.

The forceps 130 configured as described above is attached to the camera 4 when the suction cup portion 144 at the distal end portion 133 is attached to the end surface 53a of the housing 53 of the grasping unit 49 with assist functions due to the negative pressure which is generated by the operation of the air-supply/suction pump unit 140 for air suction. The camera 4 attached to the forceps 130 is introduced into the abdominal cavity 101 through the trocar 11.

In order to remove the camera 4 from the forceps 130, a positive pressure is generated by the operation of the air-supply/suction pump unit 140 for air-supply/suction, which causes the camera 4 to be removed from the suction cup portion 144. The configuration for the attachment and removal between the forceps 130 and the camera 4 based on the positive/negative pressure generated at the suction cup portion 144 may use a syringe for manual air-supply/suction, instead of the air-supply/suction pump unit 140.

Furthermore, as a configuration without the suction cup portion 144 at the distal end portion 133 of the forceps 130, as shown in Fig. 31, the camera 4 may be configured to have a projection 53c at the end surface 53a of the housing 53 of the grasping unit 49 with assist functions, and the forceps 130 may have a recess 133e at the distal end surface 133a of the distal end portion 133 to be engaged with the projection 53c of the camera 4, so that the recess 133e can be in communication with the air-supply/suction channel 143.

### (Third Modified Example)

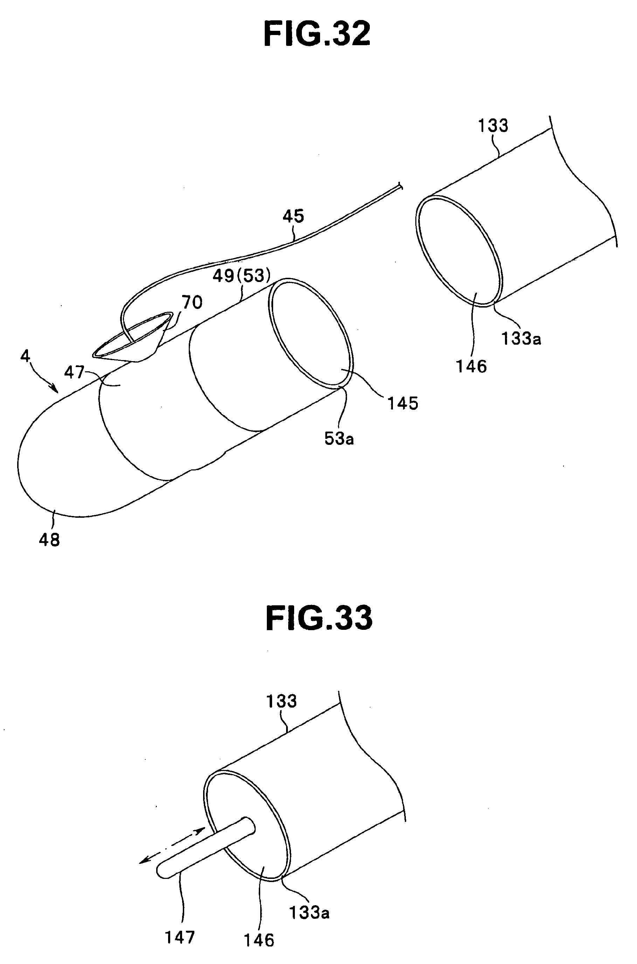

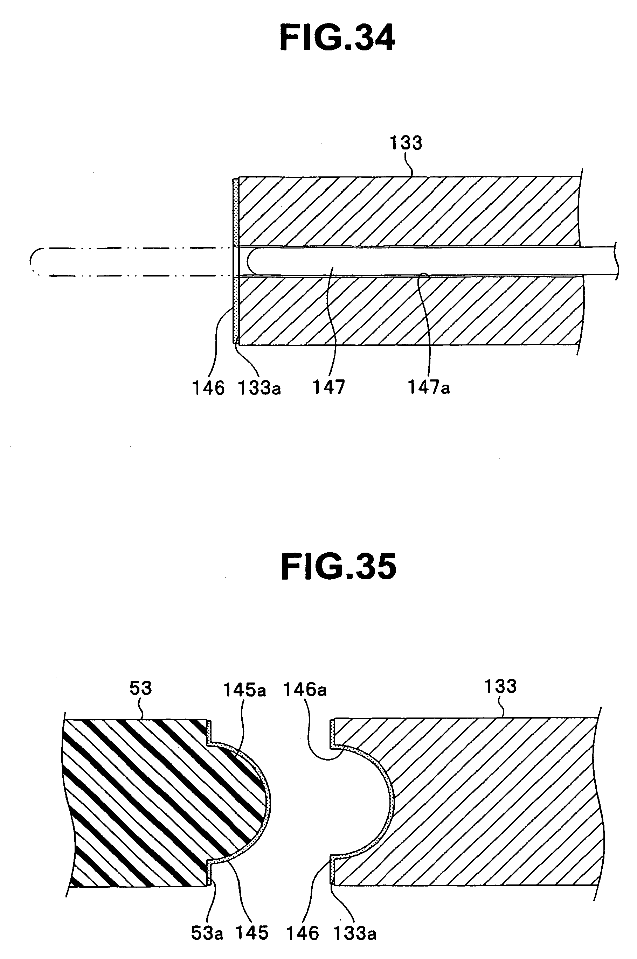

Next, a third modified example of the present embodiment will be explained below with reference to Figs. 32 to 35. Figs. 32 to 35 show the third modified example: Fig. 32 is a perspective view showing a configuration of a camera and a distal end portion of forceps; Fig. 33 is a perspective view showing a configuration of a distal end portion of forceps of another form; Fig. 34 is a sectional view showing a configuration of a distal end portion of the forceps of Fig. 33; and Fig. 35 is a sectional view showing a configuration of a housing of a camera and a distal end portion of forceps of another form.

As shown in Fig. 32, in the present modified example, adhesive layers 145 and 146 are provided to the end surface 53a of the housing 53 of the grasping unit 49 with assist functions in the camera 4 and the distal end surface 133a of the distal end portion 133 of the forceps 130, respectively. That is, a surface contact between the adhesive layer 146 of the forceps 130 and the adhesive layer 145 of the camera 4 enables the forceps 130 to adhesively hold the camera 4.

Also, as shown in Figs. 33 and 34, the forceps 130 may be configured to have a bar member 147 that protrudes from the center of the distal end surface 133a of the distal end portion 133, so that the protrusion of the bar member 147 from the distal end surface 133a causes the end surface 53a of the camera 4 to be pushed away from the distal end surface 133a, so as to remove the adhesively held camera 4 from the forceps 130. The bar member 147 is configured to be received in a hole portion 147a that is formed in the distal end portion 133 and protruded from the distal end surface 133a by an operation of the operation lever 136 of the operation portion 132 of the forceps 130, as shown in Fig. 34.

Furthermore, as shown in Fig. 35, the end surface 53a of the housing 53 of the grasping unit 49 with assist functions in the camera 4 may have a semi-spherical projection 145a that is provided with the adhesive layer 145 in the surface thereof, and a semi-spherical recess 146b provided with the adhesive layer 146 in the surface thereof may be formed in the distal end surface 133a of the distal end portion 133 of the forceps 130 to engage the projection 145a of the camera 4.

### (Fourth Modified Example)

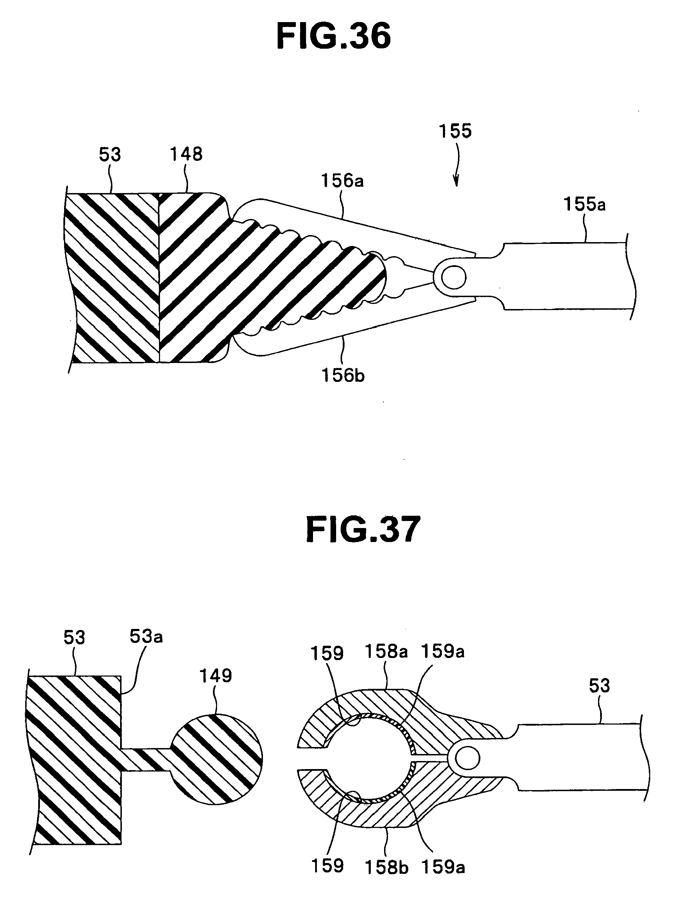

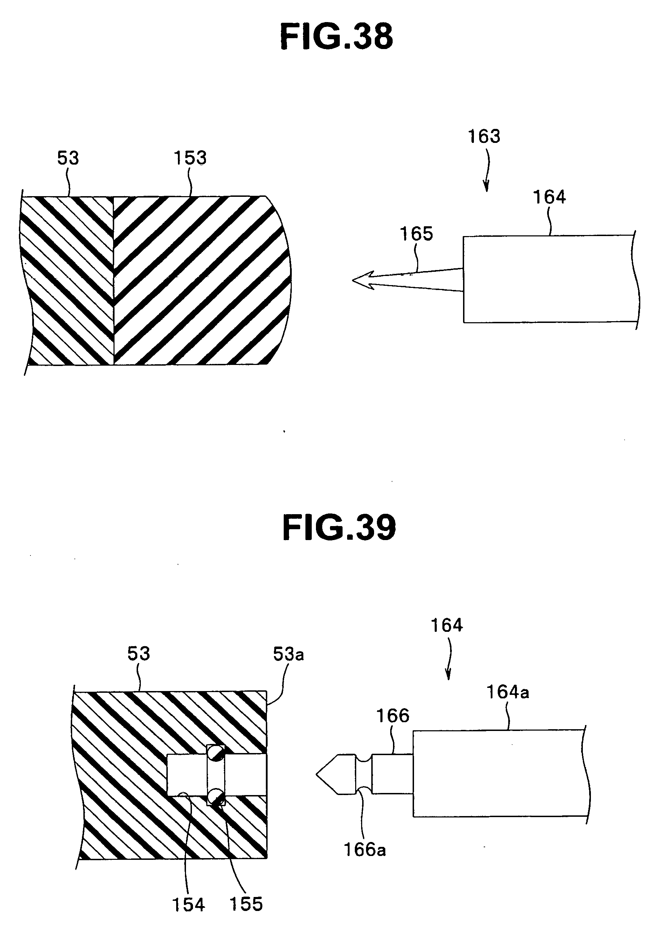

Next, a fourth modified example of the present embodiment will be explained below with reference to Figs. 36 to 39. Figs. 36 to 39 show the fourth modified example: Fig. 36 is a sectional view showing a configuration of a grasp portion at a housing of a camera which is grasped by grasping forceps; Fig. 37 is a sectional view showing a configuration of grasp portion at a housing of a camera and a distal end of forceps of another form; Fig. 38 is a sectional view showing a configuration of an elastic member at a housing of a camera and a distal end portion of forceps having a needle portion of another form; and Fig. 39 is a sectional view showing a configuration of a snap-fit structure between a housing of a camera and a distal end portion of forceps of another form.

As shown in Fig. 36, the camera 4 of the present modified example is provided with a grasp portion 148 as a grasp member that is formed of a resilient material such as sponge at the end of the housing 53 of the grasping unit 49 with assist functions.

In other words, the camera 4 is configured to be introduced and extracted in and out of a body when openable/closable jaws 156a and 156b that have teeth on the opposing sides thereof and are provided at the distal end of an insertion portion 155a of a grasping forceps 155 sandwiches the grasp portion 148 so that the teeth of the openable/closable jaws 156a and 156b engage the grasp portion 148, which allows the camera 4 to be grasped without fail. In the grasping operation, even when the grasp portion 148 is crushed by the openable/closable jaws 156a and 156b, the camera 4 can be configured not to have an increased outer diameter for grasping.

Alternatively, as shown in Fig. 37, the camera 4 is configured to be introduced and extracted in and out of a body when a spherical grasp portion 149 that is formed to be protruded at the center of the end surface 53a of the housing 53 of the grasping unit 49 with assist functions is grasped by the special grasping forceps 155.

The grasping forceps 157 includes openable/closable jaws 158a and 158b which are provided at the distal end portion of the insertion portion 157a, each of the jaws forming a semi-spherical recess 159 on the opposing side to each other. The recess 159 has generally the same curvature as that of the spherical shape of the grasp portion 149, and is provided with an elastic member 159a of a thin film such as rubber as a slip stopper on the surface thereof.

Such configuration also allows the special grasping forceps 157 to grasp the camera 4 without fail.

In the other configuration, as shown in Fig. 38, the camera 4 has a solid elastic member 153 formed of rubber or the like at the end portion of the housing 53 of the grasping unit 49 with assist functions. The camera 4 is configured to be grasped by forceps 163 that have a needle 165 at the distal end of an insertion portion 163a when the needle 165 punctures the elastic member 153. The elastic member 153 of the camera 4 has a thickness that is larger than the length of the needle 165 of the forceps 163. Thus, the entire needle 165 of the forceps 163 can puncture the elastic member 153 of the camera 4, which allows the forceps 163 to grasp the camera 4 without fail.

Furthermore, as shown in Fig. 39, the camera 4 may be configured to have a receiving portion 154 which is a hole at the center of the end surface 53a of the housing 53 of the grasping unit 49 with assist functions to be grasped by forceps 164 having a projection 166 at the distal end of an insertion portion 164a thereof when the projection 166 is fitted in the receiving portion 154. In other words, a so-called snap-fit structure can be established between the camera 4 and the forceps 164. The receiving portion 154 of the camera 4 is provided with an elastic member 154a such as C-ring and O-ring, while the projection 166 of the forceps 164 has a circumferential groove 166a around the outer peripheral portion thereof to be engaged with the elastic member 154a.

### (Fifth Modified Example)

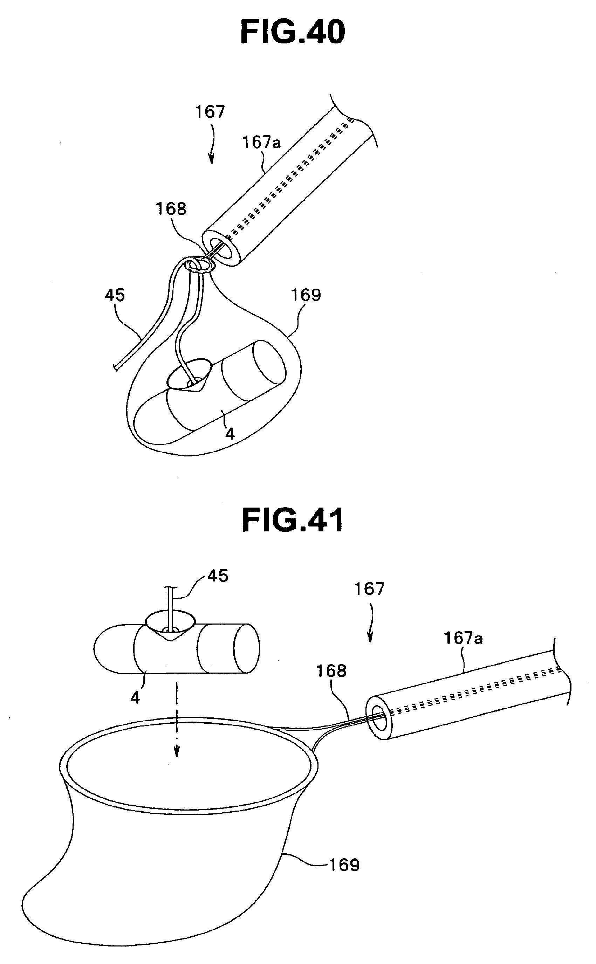

Next, a fifth modified example of the present embodiment will be explained below with reference to Figs. 40 and 41. Figs. 40 and 41 show the fifth modified example: Fig. 40 is a perspective view showing a state where a camera is already received in a bag member of forceps; and Fig. 41 is a perspective view showing a state where a camera is being received in a bag member of forceps.

As shown in Figs. 40 and 41, in the present modified example, a configuration of special forceps 167 configured to introduce and extract the camera 4 in and out of a body will be explained below.

The forceps 167 include a pipe-shaped insertion portion 167a through which an operation wire 168 is inserted. The operation wire 168 extends out of the distal end of the insertion portion 167a, and the extended portion forms a ring which is inserted into the end portion of a bag member 169 to follow almost the entire circumference of an opening of the bag member 169.

The forceps 167 is configured so that the ring is expanded/shrunk to open/close the opening of the bag member 169 in accordance with the advancing/retracting operation of the ring of the operation wire 168 that extends out of the distal end of the insertion portion 167a. In other words, the advancing/retracting operation of the operation wire 168 of the forceps 167 on the proximal end side causes the opening of the bag member 169 to be opened/closed like a so-called purse.

The camera 4 is received in the bag member 169 with the wire 45 being extended out of the closed opening of the bag member 169, when introduced in body. The wire 45 is engaged with the above described hook needle 16 (see Figs. 2 and 3) in the abdominal cavity 101 in body so that the camera 4 is pulled up to be placed against the abdominal wall 102 of the abdominal cavity 101.

Also, the camera 4 is configured to be received in the bag member 169 the opening of which is expanded, to be delivered to the outside of the body by the forceps 167, when removed from the abdominal cavity 101.

The bag member 169 is formed of a water-proof cloth-like or film-like member, which prevents any contaminant attachment of mucous membrane, blood, and the like to the camera 4 received in the bag when the camera 4 is introduced in the abdominal cavity 101. The bag member 169 is not limited to the above described member, but may be a mesh member.

### (Second Embodiment)

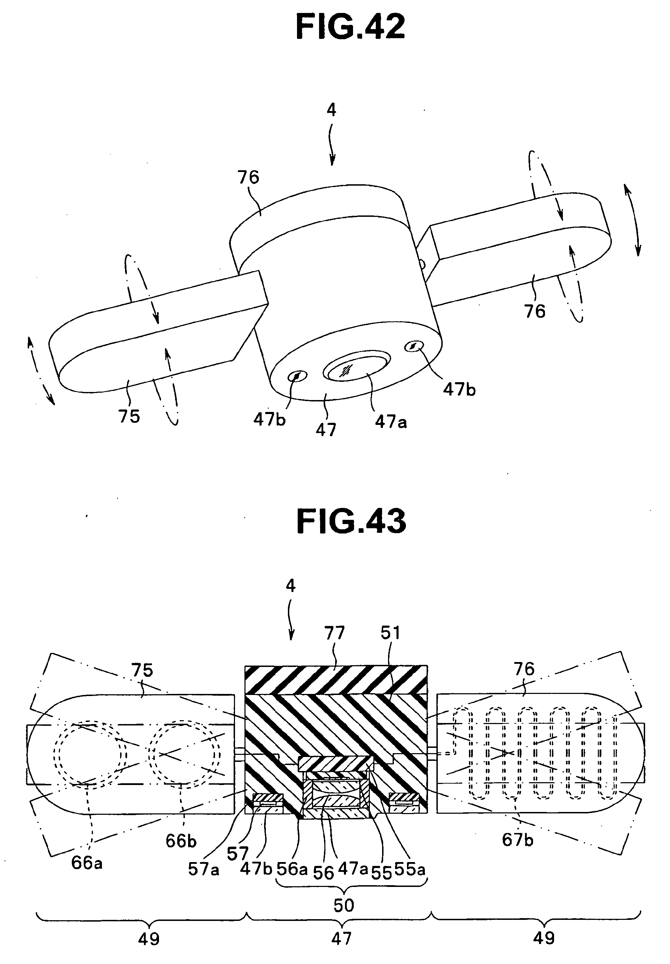

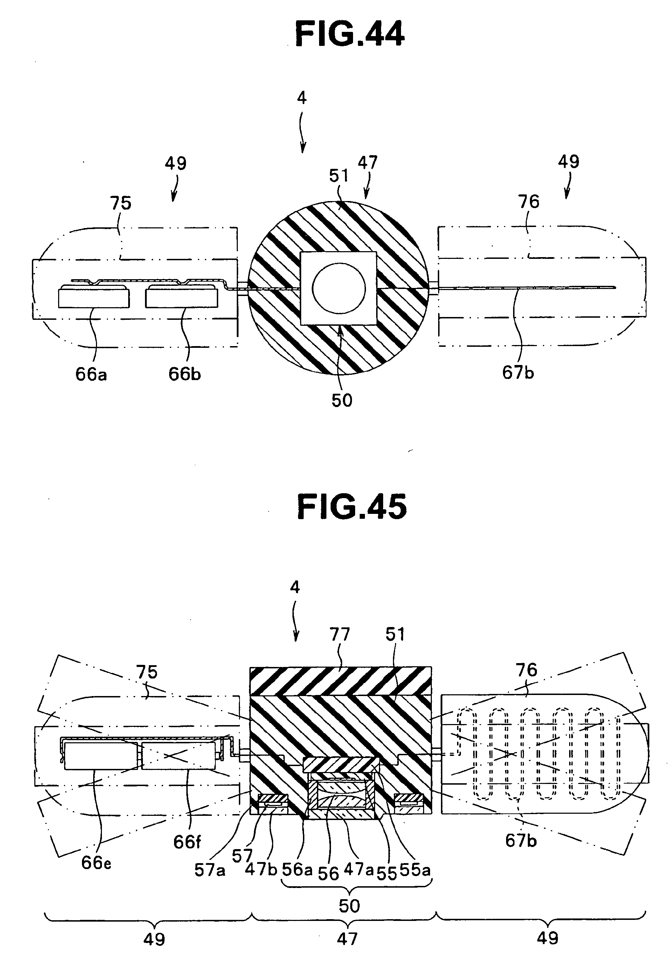

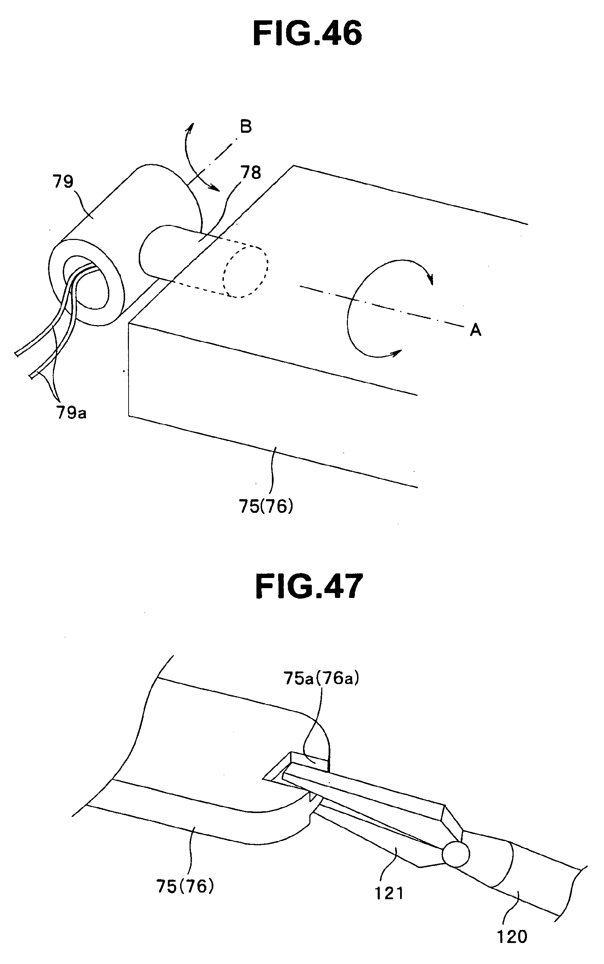

Next, with reference to Figs. 42 to 47, a second embodiment of an endoscope system according to the present disclosure will be explained below. Figs. 42 to 47 show the second embodiment of the present invention: Fig. 42 is a perspective view showing a configuration of a camera in abdominal cavity; Fig. 43 is a longitudinal sectional view showing a configuration of a camera in abdominal cavity; Fig. 44 is a transverse sectional view showing a configuration of a camera in abdominal cavity; Fig. 45 is a longitudinal sectional view showing a configuration of a camera in abdominal cavity of a modified example; Fig. 46 is a perspective view showing a configuration of a grasping unit with assist functions which is movable to a camera unit; and Fig. 47 is a perspective view showing a recess for grasping which is formed in a grasping unit with assist functions. In the following explanation, the same components as those of the endoscope system 1 according to the above described first embodiment will be denoted by the same reference numerals, the configuration of which will not be explained below in detail.

The camera 4 of the present embodiment includes the grasping units 49 with assist functions that are arranged in parallel to the camera unit 47 in the direction generally orthogonal to the viewing direction (the direction for shooting) of the image pickup unit 50 in the camera unit 47 and is rotatable about two axes relative to the camera unit 47.

Specifically, as shown in Fig. 42, the camera 4 of the present embodiment includes a camera unit 47 having a generally cylindrical outer shape, and two grasping units 49 with assist functions that are arranged at relative positions on the outer periphery portion of the camera unit 47 to be rotatable about two different axes. The two axes about which the two grasping units 49 with assist functions rotate are the two axes A and B that are generally orthogonal to each other (see Fig. 46).

The two grasping units 49 with assist functions consist of: a power source unit mounting portion 75 having two button cells 66a and 66b incorporated therein as a power source portion which is one of the assistance apparatuses; and an antenna unit mounting portion 76 having an antenna portion incorporated therein as a transmitter which is one of the assistance apparatuses, as shown in Fig. 43. The power source unit mounting portion 75 and the antenna unit mounting portion 76 have a flat shape, a so-called wing shape, that has an outer shape thinner than the camera unit 47.

The power source unit mounting portion 75 is configured to have two button cells 66a and 66b arranged therein in parallel to each other, as shown in Fig. 44, and the button cells 66a and 66b are connected to each other in parallel. This configuration allows the power source unit mounting portion 75 to be thin in the direction of the thickness thereof. The button cells 66a and 66b that constitute a power source portion may be general two AAA batteries 66e and 66f that are arranged in parallel and connected to each other in series, as shown in Fig. 45, for example. The antenna unit mounting portion 76 has the same outer shape as that of the power source unit mounting portion 75 as shown in Figs. 43 to 45, and has the antenna portion 67b meandered therein.

The power source unit mounting portion 75 and the antenna unit mounting portion 76 of the present embodiment that constitute the grasping unit 49 with assist functions include: a shaft member 78 that is arranged to extend in the longitudinal direction from the center of an end surface movably coupled to the camera unit 47; and a tube member 79 to which an extended end of the shaft member 78 is arranged to be rotatable about the axis A under a predetermined friction, as shown in Fig. 46. The tube member 79 is fitted in and held by the camera housing 51 of the camera unit 47 to be rotatable about the axis B under a predetermined friction.

Thus, the power source unit mounting portion 75 and the antenna unit mounting portion 76 that constitute the grasping unit 49 with assist functions in the present embodiment are configured to be rotatable about the axes A and B respectively, the axes A and B being orthogonal to each other, relative to the camera unit 47. Through the shaft member 78 and the tube member 79, a cable 79a passes to supply power from the button cells 66a and 66b to the image pickup unit 50 and each illumination portion 57 and each illumination drive circuit portion 57a that constitute an illumination unit, or to transmit image pickup signals from the image pickup unit 50 to the antenna portion 67b.

The camera 4 of the present embodiment further includes an abdominal wall securing portion 77 having an adhesive surface on the upper portion of the camera housing 51 in the direction opposite to the viewing direction of the image pickup unit 50. The abdominal wall securing portion 77 is configured to secure the camera 4 to an abdominal wall using the adhesion when the abdominal wall securing portion 77 is applied to a wall of the abdominal cavity. Of course, the camera 4 may be configured to have the suction cup 71 described in the first embodiment.

The power source unit mounting portion 75 and the antenna unit mounting portion 76 may have recesses 75a and 76a at the center of the front/back surfaces opposite to the camera unit 47 respectively, so that the treatment portion 121 of the treatment instrument 120 such as grasping forceps can easily grasp the camera 4.

The above described camera 4 in the endoscope system 1 of the present embodiment is configured to be rotatable about the axes A and B in the two directions orthogonal to the grasping unit 49 with assist functions, thereby especially the antenna unit mounting portion 76 can be placed at the proper angular rotational position to receive wireless transmission. The power source unit mounting portion 75 can be placed at the proper angular rotational position to prevent interference with other medical device and medical tool. The positions of the power source unit mounting portion 75 and the antenna unit mounting portion 76 that constitute the grasping unit 49 with assist functions are changed by the treatment instrument 120 such as grasping forceps to desired angular rotational positions.

Because the camera 4 has a small and low-profile configuration as in the case with the first embodiment by incorporating various assistance apparatuses in the grasping unit 49 with assist functions in parallel in the direction which is generally orthogonal to the viewing direction (the direction for shooting) of the image pickup unit 50, the assistance apparatuses being the button cells 66a and 66b or the batteries 66e and 66f as a power source portion and the antenna portion 67b of the transmitter 67, when placed in body (the abdominal cavity 101), the camera 4 is able to pick up images of a subject to be examined within a wide range of observation, and also prevents interference of itself with other medical device and medical tool that are similarly introduced in the body.

### (Third Embodiment)

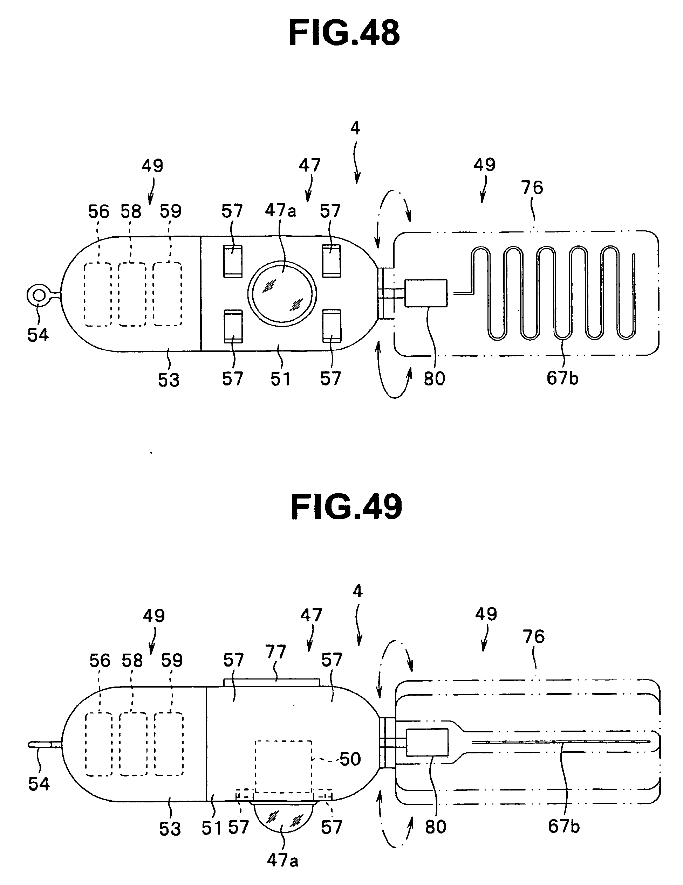

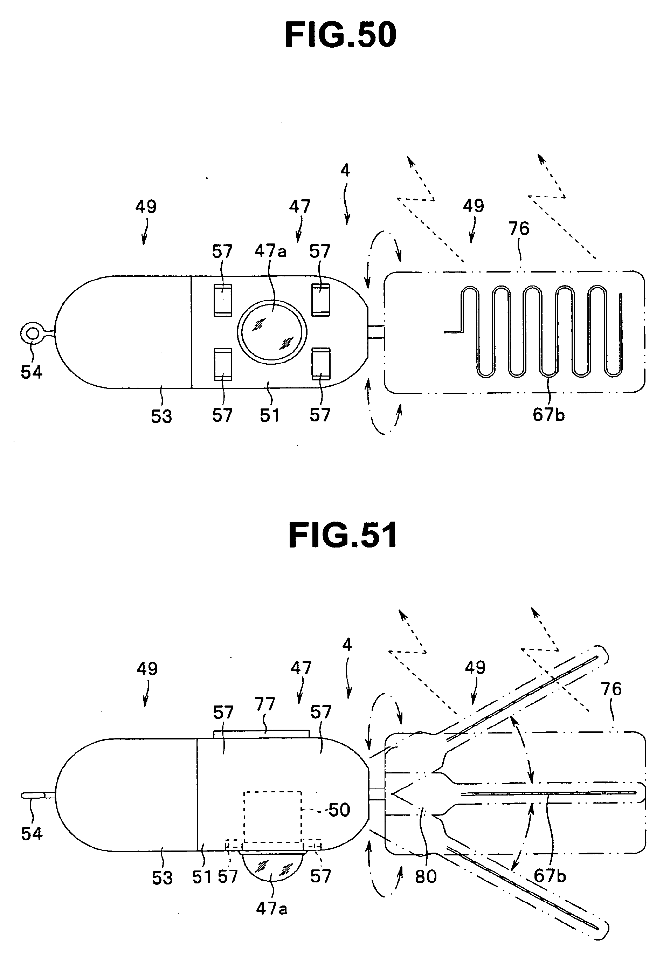

Next, with reference to Figs. 48 to 51, a third embodiment of an endoscope system according to the present disclosure will be explained below. Figs. 48 to 51 show the third embodiment of the present invention: Fig. 48 is a plan view showing a configuration of a camera in abdominal cavity; Fig. 49 is a plan view showing the camera in abdominal cavity of Fig. 48 seen from the lateral direction thereof; Fig. 50 is a plan view showing a camera in abdominal cavity of a modified example; and Fig. 51 is a plan view showing the camera in abdominal cavity of Fig. 50 seen from the lateral direction thereof. In the following explanation also, the same components as those of the camera 4 in the endoscope system 1 according to the above described first and second embodiments will be denoted by the same reference numerals, the configuration of which will not be explained below in detail.

The camera 4 of the present embodiment shown in Figs. 48 and 49 is configured to have the antenna unit mounting portion 76 which is the grasping unit 49 with assist functions described in the second embodiment. The antenna unit mounting portion 76 includes a motor 80 as a driving portion that is arranged in parallel in the direction generally orthogonal to the viewing direction (the direction for shooting) of the image pickup unit 50 of the camera unit 47. The motor 80 electrically rotates the antenna unit mounting portion 76 to a proper angular rotational position sensitive to wireless transmission relative to the camera unit 47.

The camera 4 having the motor 80 in the antenna unit mounting portion 76 may be configured so that driving of the motor 80 is stopped by a user on the outside to place the antenna unit mounting portion 76 at a proper angular rotational position sensitive to wireless transmission, or configured so that driving of the motor 80 automatically stops when the receiver 22 in the securing unit 15 (see Figs. 2 and 3) is placed at the most appropriate position sensitive to receive transmission.

In the case where the antenna unit mounting portion 76 automatically stops at the most appropriate position for the receiver 22 sensitive to receive transmission, for example, the securing unit 15 records the receiving sensitivity of the receiver 22 that receives radio waves from the antenna unit mounting portion 76 at certain time intervals to detect the rotational position where the antenna unit mounting portion 76 is the most sensitive to transmission after the antenna unit mounting portion 76 rotates once relative to the camera unit 47, so as to cause the motor 80 to stop when the antenna unit mounting portion 76 is placed at the detected angular rotational position. The information of the detected angular rotational position where the antenna unit mounting portion 76 is the most sensitive to transmission is wirelessly transmitted from securing unit 15 to the camera 4.

Alternatively, the camera 4 may be configured to control antenna unit mounting portion 76 to rotate about two axes orthogonal to the camera unit 47 so that the antenna unit mounting portion 76 stops at the most appropriate position sensitive to receive transmission, as shown in Figs. 50 and 51.

The camera 4 of the present embodiment configured as described above provides an advantage that the operation to change the angular position of the antenna unit mounting portion 76 using the treatment instrument 120 can be eliminated because the antenna unit mounting portion 76 automatically rotates, in addition to the effects described in the first and second embodiments.

### (Fourth Embodiment)

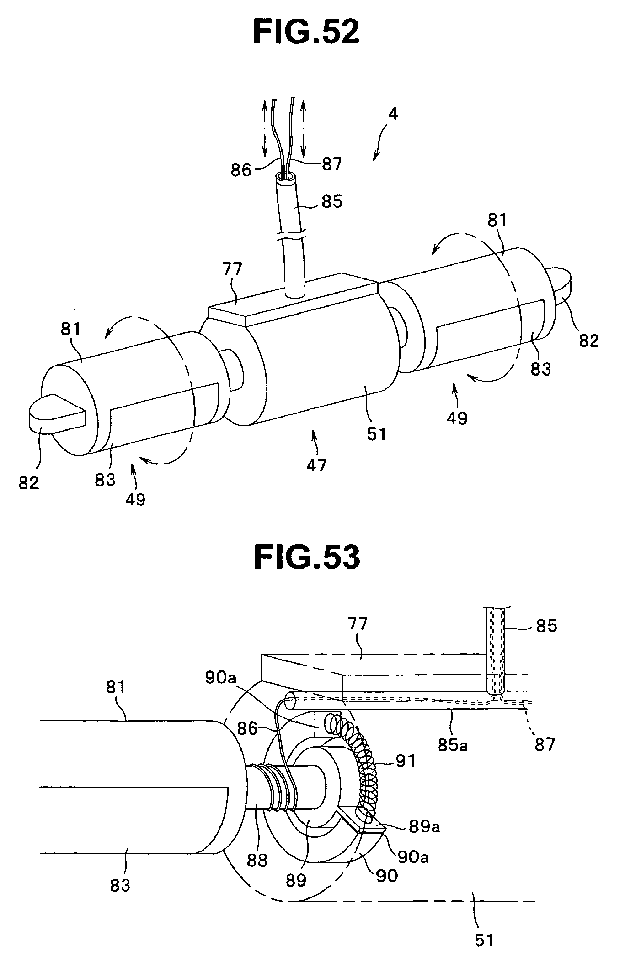

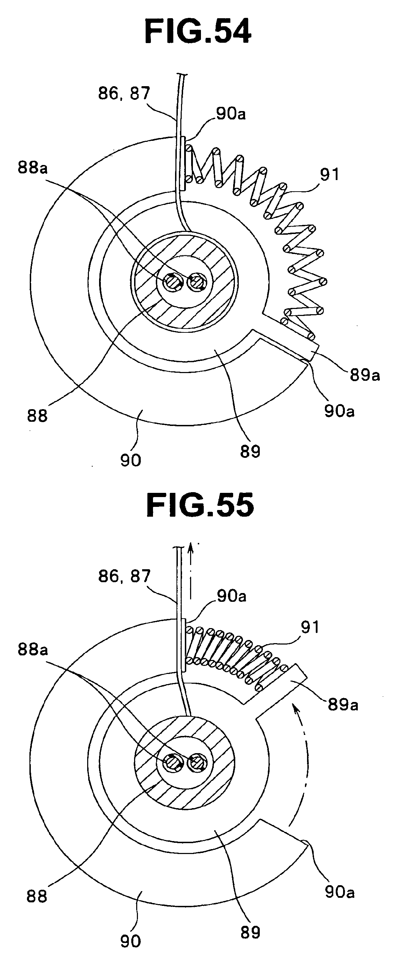

Next, with reference to Figs. 52 to 55, a fourth embodiment of an endoscope system according to the present disclosure will be explained below. Figs. 52 to 55 show the fourth embodiment of the present invention: Fig. 52 is a perspective view showing a configuration of a camera in abdominal cavity; Fig. 53 is a perspective view illustrating a configuration to cause a camera unit to rotate by an illumination unit mounting portion; Fig. 54 is a view illustrating an operation to cause a camera unit to rotate by illumination unit mounting portion; and Fig. 55 is a view illustrating an operation to cause a camera unit to rotate by illumination unit mounting portion. In the following explanation also, the same components as those of the camera 4 in the endoscope system 1 according to the above described first to third embodiments will be denoted by the same reference numerals, the configuration of which will not be explained below in detail.

The camera 4 of the present embodiment includes generally cylindrical illumination unit mounting portions 81 as the grasping unit 49 with assist functions on the both ends of the camera unit 47 that are arranged in parallel in the direction generally orthogonal to the viewing direction (the direction for shooting) of the image pickup unit 50, as shown in Fig. 52. The illumination unit mounting portions 81 include an illumination unit mounted therein with an LED or organic EL as a light source, and are arranged to be rotatably operated relative to the camera unit 47.

Each of the illumination unit mounting portions 81 has a grasp member 82 that projects out from the center of a side surface opposite to the camera unit 47. The camera 4 is introduced into a body (the abdominal cavity 101) through the trocar 111 while the grasp member 82 is sandwiched by the treatment portion 121 of the treatment instrument 120 such as grasping forceps. Each of the illumination unit mounting portions 81 further has a transparent member 83 as an illumination window through which an illumination light from the illumination unit therein is emitted to a subject to be examined. In the case where the light source for illumination light is an organic EL, the transparent member 83 is eliminated, and the organic EL may be applied to the outer surface of the illumination unit mounting portion 81 as a surface light source.

The illumination unit mounting portions 81 are rotatably operated about an axis in the longitudinal direction relative to the camera unit 47 in accordance with the drawing/relaxing operations of the operation wires 86 and 87 threaded through the tube member 85 that extends out from the center of the abdominal wall securing portion 77 on the upper portion of the camera unit 47 in the direction opposite to the viewing direction.

Next, based on Figs. 53 to 55, the configuration and operations to arrange the illumination unit mounting portion 81 to be rotatably operated relative to the camera unit 47 will be explained below.

As shown in Fig. 53, the illumination unit mounting portion 81 is rotatably supported by a shaft member 88 to be mounted to the camera housing 51 of the camera unit 47. The shaft member 88 has a cable 88a threaded therethrough to supply power to the light source (see Figs. 54 and 55), and has a generally disc-shaped rotation stopper 89 at one end thereof in the camera housing 51. The rotation stopper 89 has a tabular stopper member 89a extending outwardly from the outer periphery.

The tube member 85 is branched in the longitudinal direction of the camera housing 51 in the camera housing 51 using a branch tube 85a. The two operation wires 86 and 87 through the tube member 85 are individually directed to one of both ends of the branch tube 85a. The ends of the operation wires 86 and 87 through the tube member 85 are individually fixed to and also wound around the shaft members 88 of the illumination unit mounting portions 81 with a predetermined number of turns.

The outer periphery of the rotation stopper 89 is covered with a C-shaped securing portion 90 that is fixed to the camera housing 51, and the stopper member 89a is positioned within the notch of the securing portion 90. The securing portion 90 has two end surfaces 90a, with the upper end surface 90a being connected to one end of a compression coil spring 91. The other end of the compression coil spring 91 is fixedly attached to one surface of the stopper member 89a of the rotation stopper 89.

In the above described configuration, when the operation wires 86 and 87 are not operated for drawing and are relaxed, as shown in Fig. 54, the stopper member 89a of the rotation stopper 89 is in contact with the lower end surface (the other surface) 90a of the securing portion 90 due to the bias of the compression coil spring 91.

When the operation wires 86 and 87 wound around the shaft member 88 are operated for drawing, as shown in Fig. 55, the shaft member 88 rotates in one direction against the bias of the compression coil spring 91 that urges the stopper member 89a of the rotation stopper 89.

When the operation wires 86 and 87 are again relaxed, the compression coil spring 91 urges the stopper member 89a of the rotation stopper 89, which causes the shaft member 88 to rotate in the other direction (in the direction to return to the state of Fig. 54). In this way, the illumination unit mounting portion 81 is rotatably operated about the axis in the longitudinal direction relative to the camera unit 47 in accordance with the drawing/relaxing operations of the operation wires 86 and 87.

As described above, the camera 4 of the present embodiment provides an advantage that the illumination unit mounting portion 81 as the grasping unit 49 with assist functions is rotated about the axis in the longitudinal direction relative to the camera unit 47 in accordance with the drawing/relaxing operations of the operation wires 86 and 87 to change the illumination direction of an emitted illumination light, in addition to the effects described in above embodiments. Therefore, a user can change the illumination direction of an emitted illumination light as desired toward a subject to be examined in order to easily check the observation images obtained by the image pickup unit 50 (not shown) of the camera unit 47.

### (Fifth Embodiment)

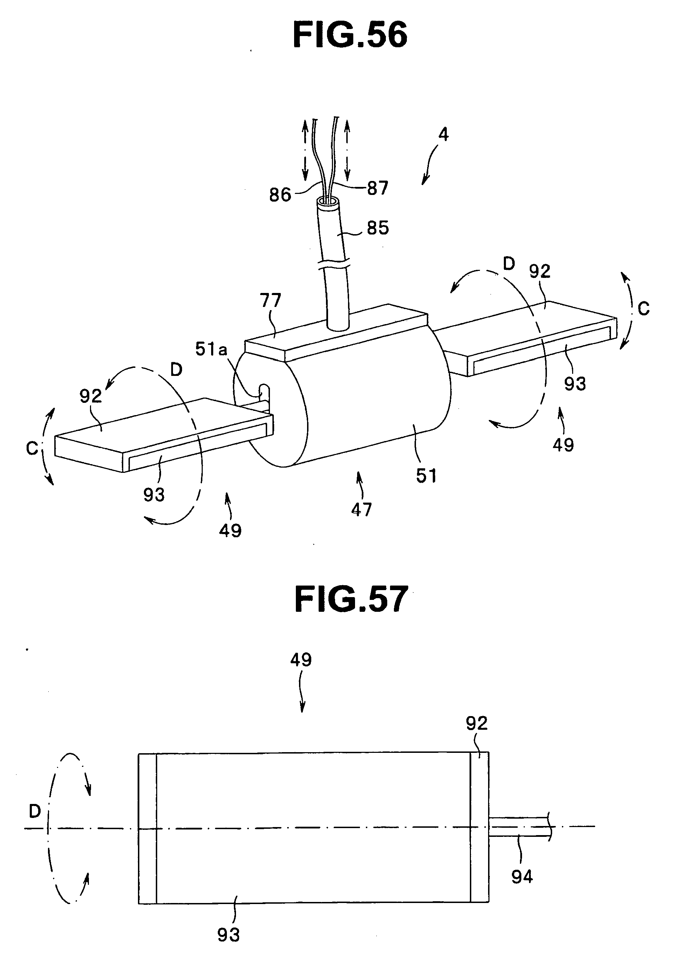

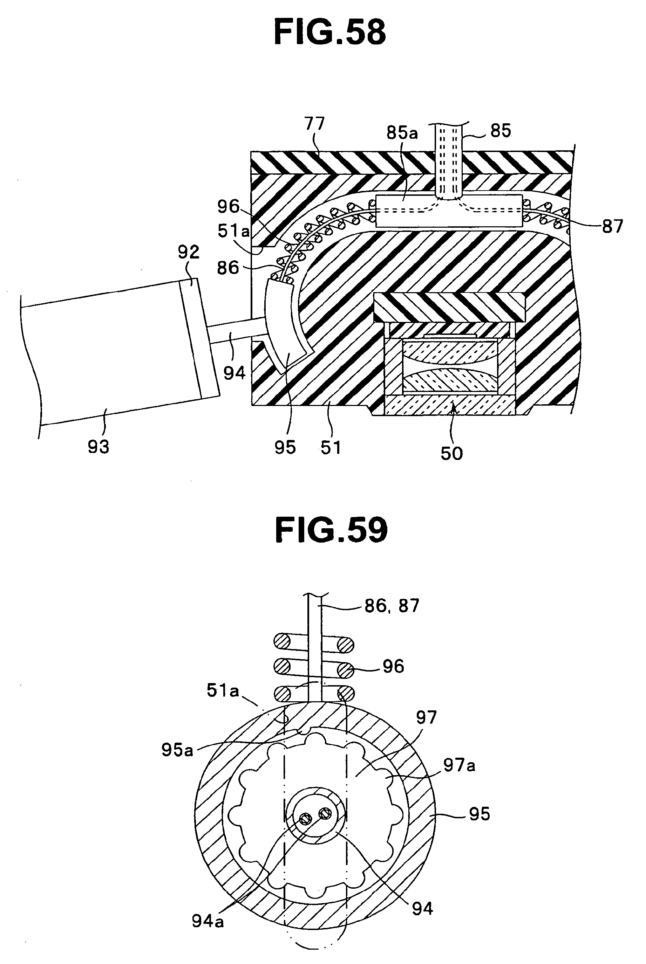

Next, with reference to Figs. 56 to 59, a fifth embodiment of an endoscope system according to the present disclosure will be explained below. Figs. 56 to 59 show the fifth embodiment of the present invention: Fig. 56 is a perspective view showing a configuration of a camera in abdominal cavity; Fig. 57 is a plan view showing a configuration of illumination unit mounting portion; Fig. 58 is a sectional view illustrating a configuration to cause a camera unit to rotate by illumination unit mounting portion; and Fig. 59 is a view illustrating a configuration of an illumination unit mounting portion that is rotatably operated about two axes. In the following explanation also, the same components as those of the camera 4 in the endoscope system 1 according to the above described first to fourth embodiments will be denoted by the same reference numerals, the configuration of which will not be explained below in detail.

The camera 4 of the present embodiment includes generally tabular illumination unit mounting portions 92 as the grasping unit 49 with assist functions that are arranged in parallel in the direction generally orthogonal to the viewing direction (the direction for shooting) of the image pickup unit 50 on the both ends of the camera unit 47, as shown in Fig. 56. The illumination unit mounting portions 92 include an illumination unit or a surface light source (transparent member 93) with an LED or organic EL as a light source therein, and are arranged to be rotatably operated relative to the camera unit 47 about two axes that are orthogonal to each other (the directions shown by the arrows C and D in Fig. 56).

The illumination unit mounting portions 92 rotate in the direction shown by the arrow C in Fig. 56 in accordance with the drawing/relaxing operations of each of the operation wires 86 and 87. While, the rotation of the illumination unit mounting portions 92 in the direction shown by the arrow D in Fig. 56 is operated by the treatment instrument 120 (not shown).

The illumination unit mounting portion 92 provides the transparent member 93 as an illumination window that has one surface inside of the illumination unit mounting portion 92, and through the illumination window, an illumination light from the illumination unit is emitted to a subject to be examined, as shown in Fig. 57. In the case where the light source for illumination light is an organic EL, the transparent member 93 is eliminated, and the organic EL may be applied to one surface of the illumination unit mounting portion 92 as a surface light source.

Next, based on Figs. 58 to 59, the configuration and operations to arrange the illumination unit mounting portion 92 to be rotatably operated relative to the camera unit 47 about two axes will be explained below.

As shown in Figs. 58 and 59, the illumination unit mounting portions 92 individually have a shaft member 94 extending outwardly from the center of one surface thereof. The shaft member 94 has a cable 94a threaded therethrough to supply power to the light source.

The shaft member 94 is provided with a hollow disk member 95 at the extended end thereof. The disk member 95 is movably supported in a guide hole portion 51 a that is formed in the camera housing 51 of the camera unit 47 and has a circular cross section, and is arranged in the camera unit 47 not to slip down so as to allow the illumination unit mounting portions 92 to rotate about an axis in one direction (the direction shown by the arrow C in Fig. 56). Each of the disk member 95 is connected with one end of the operation wires 86 or 87 at a part of the outer periphery thereof.

The operation wires 86 and 87 are covered with a compression coil spring 96 in the guide hole portion 51 a. The compression coil spring 96 is fixedly attached to the end surface of the branch tube 85a at one end thereof, and is arranged in the guide hole portion 51 a to be in contact with the outer peripheral surface of the disk member 95 at the other end thereof.

In other words, when the operation wire 86 (87) is drawn, the shaft member 94 of the illumination unit mounting portion 92 is drawn along the guide hole portion against the bias of the compression coil spring 96 that urges the disk member 95. This allows the illumination unit mounting portion 92 to rotate about an axis in one direction (the direction shown by the arrow C in Fig. 56) relative to the camera unit 47. The camera unit 47 has an opening of the guide hole portion 51 a having a long hole shape formed in one side surface thereof, so that the shaft member 94 of the illumination unit mounting portion 92 is guided along the opening of the guide hole portion 51a.

The disk member 95 holds the shaft member 94 in a rotatable manner. The shaft member 94 has an outwardly extending flange 97 at one end in the disk member 95. The outwardly extending flange 97 is provided with a plurality of projection 97a along the outer peripheral portion thereof at equal intervals. The disk member 95 is provided with a projection 95a on the inner peripheral surface thereof, which contacts with one of the plurality of projection 97a while the shaft member 94 is rotating.

Because the illumination unit mounting portion 92 includes the shaft member 94 that is rotatably arranged relative to the disk member 95, the illumination unit mounting portion 92 is configured to be rotatably operated about an axis in another direction (the direction shown by the arrow D in Fig. 56) relative to the camera unit 47 by the treatment instrument 120 (not shown) such as grasping forceps.

In addition, because the projection 95a on the inner surface of the disk member 95 contacts with one of the plurality of projection 97a on the outer peripheral portion of the shaft member 94, the illumination unit mounting portion 92 can be fixed at a rotational stepwise position a user desires. The projections 95a and 97a have a height that is set so that the plurality of projections 97a on the outer peripheral portion of the shaft member 94 are able to go beyond the projection 95a on the inner surface of the disk member 95 with a predetermined rotational force.

As described above, the camera 4 of the present embodiment also provides an advantage, in addition to the effects described in above embodiments, that the direction of illumination light is freely changeable because the illumination unit mounting portion 92 as the grasping unit 49 with assist functions is rotatably operated about an axis in the lateral direction (the direction shown by the arrow C in Fig. 56) relative to the camera unit 47 by the drawing/relaxing operations of the operation wires 86 and 87, and also is rotatably operated about another axis in the longitudinal direction (the direction shown by the arrow D in Fig. 56) by the treatment instrument 120. Therefore, a user can change the illumination direction of an emitted illumination light as desired toward a subject to be examined more freely than the case in the fourth embodiment in order to easily check the observation images obtained by the image pickup unit 50 (not shown) of the camera unit 47.

### (Sixth Embodiment)

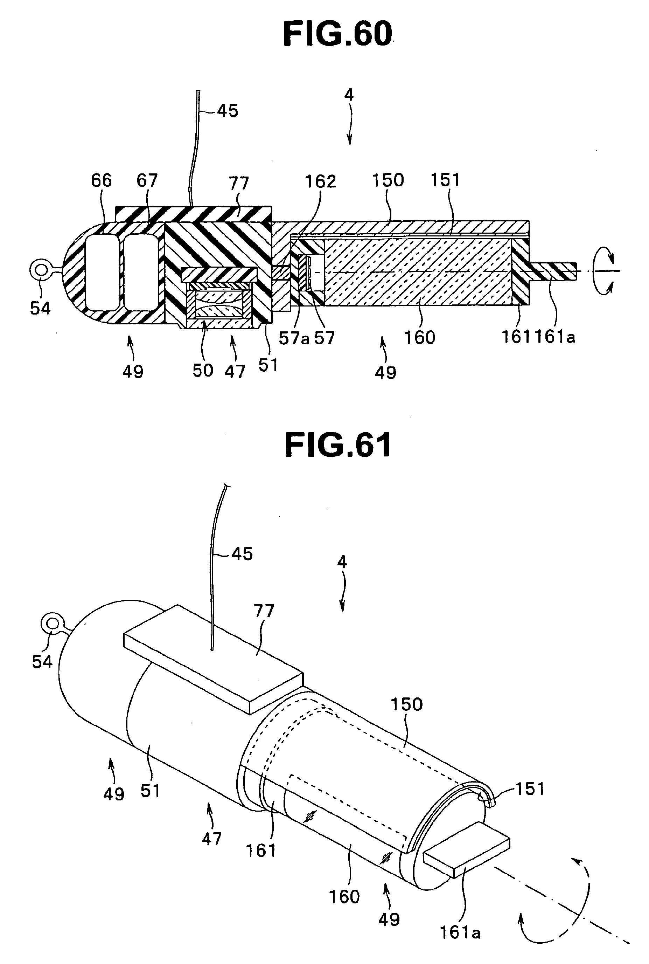

Next, with reference to Figs. 60 and 61, a sixth embodiment of an endoscope system according to the present disclosure will be explained below. Figs. 60 and 61 show the sixth embodiment of the present invention: Fig. 60 is a sectional view showing a configuration of a camera in abdominal cavity; and Fig. 61 is a perspective view showing a configuration of a camera in abdominal cavity. In the following explanation also, the same components as those of the endoscope system 1 according to the above described first to fifth embodiments will be denoted by the same reference numerals, the configuration of which will not be explained below in detail.

As shown in Figs. 60 and 61, in the camera 4 of the present embodiment, one grasping unit 49 with assist functions is configured with an illumination unit including the illumination portion 57 and the illumination drive circuit portions 57a. The grasping unit 49 with assist functions is arranged in parallel in a direction generally orthogonal to the viewing direction (the direction for shooting) of the image pickup unit 50 of the camera unit 47, and is rotatable about an axis in the longitudinal direction relative to the camera unit 47.

As a specific configuration, the grasping unit 49 with assist functions having the illumination unit is generally configured with a reflecting hood 150 that has a generally semicircular shape and has a reflecting film 151 on the inner surface thereof, and a rotary frame 161 that incorporates the illumination portion 57 and the illumination drive circuit portions 57a therein and includes an illumination light guiding member 160 having a generally cylindrical shape. The rotary frame 161 is fitted in the reflecting hood 150, and both are rotatable about a support shaft 162 which is fixed to one side surface of the camera unit 47.

The illumination portion 57 and the illumination drive circuit portions 57a in the rotary frame 161 are arranged to emit illumination light toward the illumination light guiding member 160. The illumination light guiding member 160 diffuses the incident illumination light in the direction toward the outer peripheral portion thereof, so that the brightness and distribution of the illumination light to a subject to be examined is improved.

The rotary frame 161 also has a tabular grasping member 161a on one end surface thereof which is configured to be grasped by the treatment instrument 120 (not shown) such as grasping forceps.

The reflecting hood 150 covers about one half of the outer peripheral portion of the illumination light guiding member 160, and reflects the illumination light emitted from the outer peripheral portion of the illumination light guiding member 160 by the reflecting film, so that the illumination light is directed to a subject to be examined.

In the camera 4 of the present embodiment configured as described above, the grasping member 161 a of the rotary frame 161 is sandwiched between the treatment instrument 120 (not shown) such as grasping forceps, and the reflecting hood 150 and the rotary frame 161 are rotatably operated about an axis in the longitudinal direction relative to the camera unit 47. This configuration allows a user to direct an illumination light to a subject to be examined for observation as desired. Furthermore, although the light source of the illumination unit has a small area, because the camera 4 includes the illumination light guiding member 160 in the grasping unit 49 with assist functions, any light from the light source can be efficiently reflected and the brightness and distribution of the light can be improved as an illumination light.

A rotary switch may be provided between the camera unit 47 and the rotary frame 161, so that the rotary frame 161 can be operated to stepwisely change the brightness of light from the illumination unit. As a result, a brightness of an illumination light can be controlled to be proper to shooting by the image pickup unit 50.

### (Seventh Embodiment)

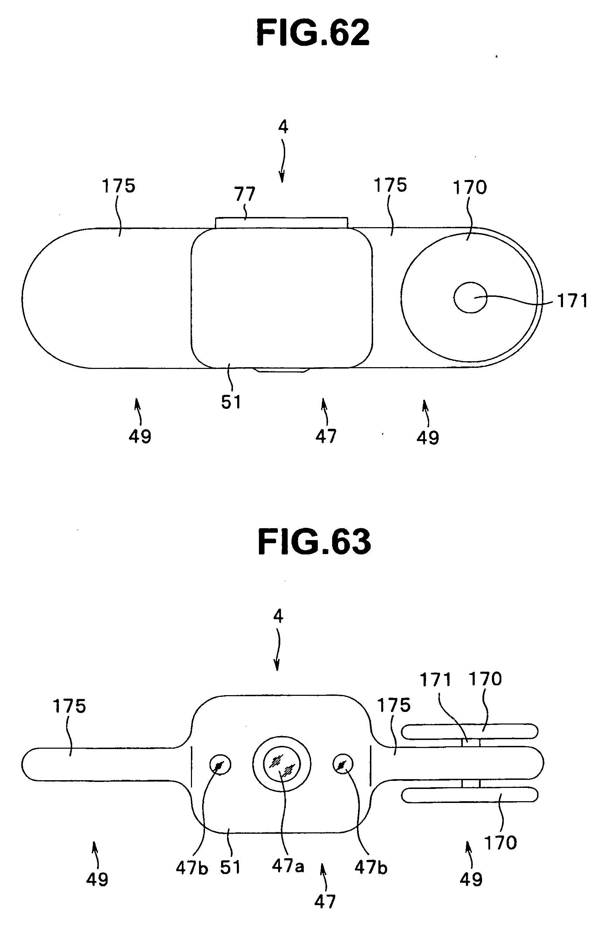

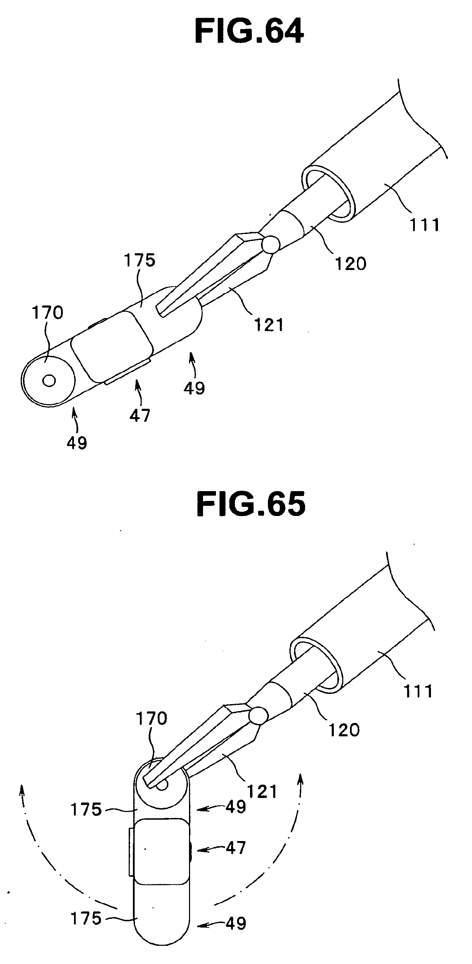

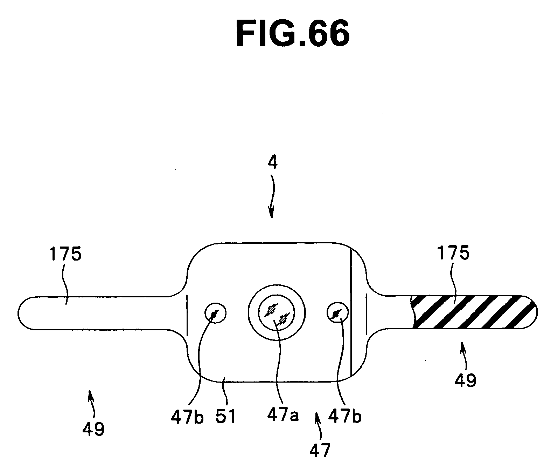

Next, with reference to Figs. 62 to 66, a seventh embodiment of an endoscope system according to the present disclosure will be explained below. Figs. 62 to 66 show the seventh embodiment of the present invention: Fig. 62 is a plan view showing a configuration of a camera in abdominal cavity; Fig. 63 is a plan view showing the camera in abdominal cavity of Fig. 62 seen from the direction toward an observation window and an illumination window provided to the camera; Fig. 64 is a view illustrating an operation to cause a camera in abdominal cavity to be introduced into an abdominal cavity using a treatment instrument; Fig. 65 is a view illustrating an operation to cause a camera in abdominal cavity to be removed from the abdominal cavity to the outside of the body through a trocar using a treatment instrument; and Fig. 66 is a plan view showing the camera in abdominal cavity of a modified example seen from the direction toward an observation window and an illumination window provided to the camera. In the following explanation also, the same components as those of the endoscope system 1 according to the above described first to sixth embodiments will be denoted by the same reference numerals, the configuration of which will not be explained below in detail.

The camera 4 of the present embodiment is configured with two circular plate grasp members 170 on the front and back surfaces of one housing frame 175 of the two the grasping units 49 with assist functions that are arranged in parallel in the direction generally orthogonal to the viewing direction (the direction for shooting) of the image pickup unit 50 of the camera unit 47, so as to be integrally rotatable, as shown in Figs. 62 and 63.

The two circular plate grasp members 170 are coupled to each other with the one housing frame 175 being sandwiched therebetween, and a shaft member 171 is rotatably provided to the housing frame 175. Thus, the circular plate grasp members 170 are arranged to one housing frame 175 to be rotatable about the shaft member 171 relative to the housing frame 175. The housing frame 175 is formed of a rigid member.

The camera 4 configured as described above is grasped by the treatment portion 121 of the treatment instrument 120 such as grasping forceps as shown in Fig. 64 on the housing frame 175 side that is not provided with the circular plate grasp member 170, when introduced into the abdominal cavity of a subject through the trocar 111. In other words, the camera 4 is introduced into the abdominal cavity of a subject through the trocar 111 while being grasped by the treatment instrument 120 such as grasping forceps in an immobilized state.

While, when the camera 4 is removed from the abdominal cavity of a subject after surgery, as shown in Fig. 65, the two circular plate grasp members 170 are grasped by the treatment portion 121 of the treatment instrument 120 such as grasping forceps. In the state, the two circular plate grasp members 170 are immobilized by the treatment instrument 120, but the other parts of the camera 4 are freely rotatable due to the two circular plate grasp members 170.

This allows the camera 4 to be easily introduced into the trocar 111 without being trapped, even if the camera 4 abuts the opening of the trocar 111 when removed to the outside of the body through the trocar 111.

As described above, the camera 4 of the present embodiment may have a configuration that provides the above described effects and improves the removability from the abdominal cavity of a subject after surgery. Even when the camera 4 is deformably configured with one housing frame 175 formed of a soft elastic material such as rubber, without the two circular plate grasp member 170 as shown in Fig. 66, the same effects can be obtained.

## Claims

1. A medical apparatus having a camera (4) that is introduced into a body, wherein the camera (4) is a capsule-type image pickup unit comprising:
an image pickup unit (50) that is provided with a camera housing (51) of a camera unit (47), and that is configured to pick up an image of a subject to be examined in the body;
a securing portion (70) configured to secure the camera (4) to a wall in the body, the securing portion (70) being removably disposed on an outer peripheral surface of the camera housing (51) of the camera unit (47) and opposite to a viewing direction of the image pickup unit (50) and having a trough hole (72) formed centraly thereof;
a grasping unit (49) that constitutes one side end portion of the camera (4) in a longitudinal direction thereof and is removably arranged on a first side of the camera housing (51) of the camera unit (47) in a row arrangement;
a plurality of assistance apparatuses (58, 59, 66, 66a-f, 67, 68) incorporated into the grasping unit (49) and configured to assist image pickup by the image pickup unit (50)
a grasp portion (54) disposed at a center of an end surface of the grasping unit (49) and configured to be grasped when the camera (4) is introduced into and retracted out of the body;
a wire (45) inserted through the hole (72) and configured to lift the camera (4) while bringing the securing portion (70) into close contact with the wall in the body, to secure the camera (4) to the wall, the wire having one end connected to the camera housing (51) and configured to be extended from a center portion of the securing portion (70); and
a balancing member (48) that constitutes a second side end portion of the camera (4) and is removably arranged on a second side of the camera housing (51) of the camera unit (47) opposite to the fist side of the camera housing (51) in a row arrangement, the balancing member (48) being configured to stabilize a balance for securing of the camera (4) when the camera (4) is secured to the wall, and designed to have the same weight as the grasping unit (49) in which the plurality of assistance apparatuses (58, 59, 66, 66a-f, 67, 68) are incorporated.

2. The medical apparatus according to claim 1, wherein each of the plurality of assistance apparatuses (58, 59, 66, 66a-f, 67, 68) are incorporated in the grasping unit (49) in parallel along the longitudinal direction of the camera (4) on one side of the image pickup portion (47, 50).

3. The medical apparatus according to claim 1 or 2, wherein the assistance apparatus (58, 59, 66, 66a-f, 67, 68) at least includes a power source portion (66, 66a-f) configured to drive the image pickup portion (47, 50), and an antenna (67b) configured to wirelessly transmit image data obtained from the image pickup portion (47, 50) to an external device (3).

4. The medical apparatus according to any one of claims 1 to 3, wherein the assistance apparatus (58, 59, 66, 66a-f, 67, 68) includes an illumination unit (57) configured to emit an illumination light to the subject to be examined.

## Patentansprüche

1. Medizinisches Gerät mit einer Kamera (4), die in einen Körper eingeführt ist, wobei die Kamera (4) eine Kapsel-Bildaufnahmeeinheit ist, welche umfasst:
eine Bildaufnahmeeinheit (50), die mit einem Kameragehäuse (51) einer Kameraeinheit (47) versehen ist und die dazu eingerichtet ist, ein Bild eines zu untersuchenden Subjekts in dem Körper aufzunehmen;
einen Sicherungsabschnitt (70), der dazu eingerichtet ist, die Kamera (4) an einer Wand in dem Körper zu sichern, wobei der Sicherungsabschnitt (70) lösbar an einer Außenumfangsfläche des Kameragehäuses (51) der Kameraeinheit (47) und entgegengesetzt zu einer Beobachtungsrichtung der Bildaufnahmeeinheit (50) angeordnet ist und eine zentral ausgebildete Durchgangsöffnung (72) hat;
eine Greifeinheit (49), die einen Endseitenabschnitt der Kamera (4) in ihrer Längsrichtung bildet und lösbar an einer ersten Seite des Kameragehäuses (51) der Kameraeinheit (47) in einer Reihenanordnung angeordnet ist;
eine Mehrzahl von Unterstützungsgeräten (58, 59, 66, 66a-f, 67, 68), die in die Greifeinheit (49) integriert und dazu eingerichtet sind, die Bildaufnahme durch die Bildaufnahmeeinheit (50) zu unterstützen;
einen Greifabschnitt (54), der an einem Mittelpunkt einer Endoberfläche der Greifeinheit (49) angeordnet und dazu eingerichtet ist, gegriffen zu werden, wenn die Kamera (4) in den Körper eingeführt und daraus zurückgezogen wird;
einen Draht (45), der durch die Öffnung (72) eingeführt und dazu eingerichtet ist, die Kamera (4) anzuheben, während der Sicherungsabschnitt (70) in engen Kontakt mit der Wand in dem Körper gebracht wird, um die Kamera (4) an der Wand zu sichern, wobei der Draht ein Ende hat, das mit dem Kameragehäuse (51) verbunden und dazu eingerichtet ist, sich von einem zentralen Abschnitt des Sicherungsabschnitts (70) zu erstrecken; und
ein Gleichgewichtselement (48), das einen zweiten Seitenendabschnitt der Kamera (4) bildet und lösbar an einer zweiten Seite des Kameragehäuses (51) der Kameraeinheit (47) entgegengesetzt zu der ersten Seite des Kameragehäuses (51) in einer Reihenanordnung angeordnet ist, wobei das Gleichgewichtselement (48) dazu eingerichtet ist, ein Gleichgewicht zum Sichern der Kamera (4) zu stabilisieren, wenn die Kamera (4) an der Wand gesichert wird, und so konfiguriert ist, dass es das gleiche Gewicht hat wie die Greifeinheit (49), in die die Mehrzahl von Unterstützungsgeräten (58, 59, 66, 66a-f, 67, 68) integriert ist.

2. Medizinisches Gerät nach Anspruch 1, bei dem jedes der Mehrzahl von Unterstützungsgeräten (58, 59, 66, 66a-f, 67, 68) in die Greifeinheit (49) parallel entlang der Längsrichtung der Kamera (4) auf einer Seite des Bildaufnahmeabschnitts (47, 50) integriert ist.

3. Medizinisches Gerät nach Anspruch 1 oder 2, bei dem das Unterstützungsgerät (58, 59, 66, 66a-f, 67, 68) zumindest einen Energiequellenabschnitt (66, 66a-f), der dazu eingerichtet ist, den Bildaufnahmeabschnitt (47, 50) anzutreiben, und eine Antenne (67b) umfasst, die dazu eingerichtet ist, von dem Bildaufnahmeabschnitt (47, 50) erhaltene Bilddaten drahtlos an eine externe Einrichtung (3) zu übertragen.

4. Medizinisches Gerät nach einem der Ansprüche 1 bis 3, bei dem das Unterstützungsgerät (58, 59, 66, 66a-f, 67, 68) eine Beleuchtungseinheit (57) umfasst, die dazu eingerichtet ist, ein Beleuchtungslicht auf das zu untersuchende Subjekt zu emittieren.

## Revendications

1. Appareil médical ayant une caméra (4) qui est introduit dans un corps, dans lequel la caméra (4) est une unité de capture d'image de type capsule comprenant :
une unité (50) de capture d'image qui est prévue avec un logement (51) de caméra d'une unité (47) de caméra, et qui est configurée pour capturer une image d'un sujet devant être examiné dans le corps ;
une partie (70) de fixation configurée pour fixer la caméra (4) à une paroi dans le corps, la partie (70) de fixation étant disposée amovible sur une surface périphérique extérieure du logement (51) de caméra de l'unité (47) de caméra, et opposée à un sens de vue de l'unité (50) de capture d'image et ayant un trou traversant (72) formé centralement dans celle-ci ;
une unité (49) de préhension qui constitue une partie d'extrémité latérale de la caméra (4) dans un sens longitudinal de celle-ci et est agencée amovible sur un premier côté du logement (51) de caméra de l'unité (47) de caméra dans un agencement en rangée ;
une pluralité d'appareils (58, 59, 66, 66a-f, 67, 68) d'assistance incorporés dans l'unité (49) de préhension et configurés pour aider à une capture d'image par l'unité (50) de capture d'image ;
une partie (54) de préhension disposée en un centre d'une surface d'extrémité de l'unité (49) de préhension et configurée pour être prise lorsque la caméra (4) est introduite dans le et retirée hors du corps ;
un fil (45) inséré à travers le trou (72) et configuré pour lever la caméra (4) tout en amenant la partie (70) de fixation en contact étroit avec la paroi dans le corps, pour fixer la caméra (4) à la paroi, le fil ayant une extrémité connectée au logement (51) de caméra et configuré pour être étendu depuis une partie centrale de la partie (70) de fixation ; et
un élément (48) d'équilibrage qui constitue une deuxième partie d'extrémité latérale de la caméra (4) et est agencé amovible sur un deuxième côté du logement (51) de caméra de l'unité (47) de caméra opposé au premier côté du logement (51) de caméra dans un agencement en rangée, l'élément (48) d'équilibrage étant configuré pour stabiliser un équilibre pour la fixation de la caméra (4) lorsque la caméra (4) est fixée à la paroi, et conçu de façon à avoir le même poids que l'unité (49) de préhension dans laquelle la pluralité d'appareils (58, 59, 66, 66a-f, 67, 68) d'assistance sont incorporés.

2. Appareil médical selon la revendication 1, dans lequel chacun de la pluralité d'appareils (58, 59, 66, 66a-f, 67, 68) d'assistance est incorporé dans l'unité (49) de préhension en parallèle le long du sens longitudinal de la caméra (4) sur un côté de la partie (47, 50) de capture d'image.

3. Appareil médical selon la revendication 1 ou 2, dans lequel les appareils (58, 59, 66, 66a-f, 67, 68) d'assistance incluent au moins une partie (66, 66a-f) de source d'énergie configurée pour entraîner la partie (47, 50) de capture d'image, et une antenne (67b) configurée pour transmettre sans fil des données d'image obtenues de la partie (47, 50) de capture d'image à un dispositif externe (3).

4. Appareil médical selon l'une quelconque des revendications 1 à 3, dans lequel les appareils (58, 59, 66, 66a-f, 67, 68) d'assistance incluent une unité (57) d'éclairage configurée pour émettre une lumière d'éclairage sur le sujet devant être examiné.
